(19) Europäisches Patentamt
European Patent Office
Office européen des brevets

(11) Veröffentlichungsnummer: **0 262 393 B1**

(12) **EUROPÄISCHE PATENTSCHRIFT**

(45) Veröffentlichungstag der Patentschrift: **07.01.93**

(21) Anmeldenummer: **87112370.9**

(22) Anmeldetag: **26.08.87**

(51) Int. Cl.5: **C07C 255/29**, C07C 255/31, C07C 255/57, C07C 235/20, C07C 323/23, C07C 323/01, C07D 213/65, C07D 215/26, C07D 239/36, A01N 37/34

# (54) Aryloxycarbonsäurederivate, ihre Herstellung und Verwendung.

(30) Priorität: **29.08.86 DE 3629441**
**30.01.87 DE 3702964**

(43) Veröffentlichungstag der Anmeldung:
**06.04.88 Patentblatt 88/14**

(45) Bekanntmachung des Hinweises auf die Patenterteilung:
**07.01.93 Patentblatt 93/01**

(84) Benannte Vertragsstaaten:
**AT BE CH DE ES FR GB GR IT LI LU NL SE**

(56) Entgegenhaltungen:
**EP-A- 0 001 721**
**EP-A- 0 003 261**
**DE-A- 2 622 977**
**DE-A- 3 001 433**
**DE-B- 2 711 738**
**US-A- 3 932 168**
**US-A- 4 001 427**
**US-A- 4 087 277**
**US-A- 4 119 433**

**CHEMICAL ABSTRACTS, Band 62, Nr. 4, 15. Februar 1965, Spalte 2, Zusammenfassungsnr. 4106, Columbus, Ohio, US; G.A. MASLOVA et al.: "Alpha-Phenoxyacylaminocarboxylic acids and their derivatives", & ZH. OBSHCH. KHIM. 1964, 34(10), 3411-3414**

(73) Patentinhaber: **SHELL INTERNATIONALE RESEARCH MAATSCHAPPIJ B.V.**
**Carel van Bylandtlaan 30**
**NL-2596 HR Den Haag(NL)**

(72) Erfinder: **Buck, Wolfgang, Dr.**
**In der Dörrwiese 37**
**W-6507 Ingelheim am Rhein(DE)**
Erfinder: **Raddatz, Erich, Dr.**
**Carrera 1-Oe No. 5265**
**Cali(CO)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft neue Mittel zur Bekämpfung phytopathogener Pilze, neue Wirkstoffe für diese Mittel und verfahren zur Herstellung der Wirkstoffe.

EP-A-1721 offenbart gewisse Verbindungen mit der Formel:

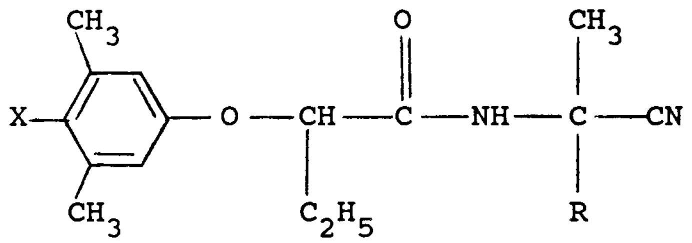

(A)

wo X ein Chlor- oder Bromatom oder eine Methylgruppe und R eine Methyl-, Ethyl- oder Methoxymethylgruppe bedeutet. Die Anwendung solcher Verbindungen in der Bekämpfung von Mehltau, insbesondere Bohnenmehltau (Erysiphe polygoni), wird ebenfalls offenbart.

Es ist aber nun entdeckt worden, dass gewisse andere Derivate von Aryloxy- und Arylthiocarbonsäure für die Bekämpfung phytopathogener Pilze nützlich sind, insbesondere Piricularia an Reispflanzen.

Erfindungsgemäss wird deshalb die Verwendung von Verbindungen vorgesehen, die der Formel I.

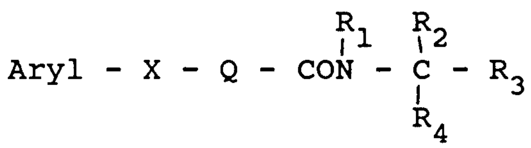

(I)

entsprechen, in der

Aryl: einen unsubstituierten oder ein- bis dreifach durch $C_1$-$C_5$-Alkyl, $C_1$-$C_5$-Alkoxy, $C_1$-$C_5$-Alkyl-$SO_n$ (n = 0, 1 oder 2), Halogen, $NO_2$, $CF_3$, CN, $CH_3OCH_2$, $(CH_3)_2NCH_2$, COOAlkyl, $CONH_2$ oder Phenyl substituierten Phenylrest, einen 1- oder 2-Naphthyl-, einen gegebenenfalls chlorsubstituierten 2-, 3- oder 4-Pyridyl-, einen Pyrimidyl oder Chinolylrest,

Q:

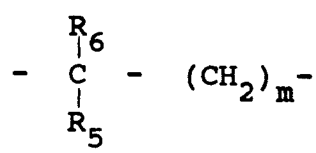

(m = 0,1 oder 2)

$R_1$: H, $C_1$-$C_5$-Alkyl, Allyl,

$R_2$ und $R_3$: H, $C_1$-$C_6$-Alkyl (das auch ein 0- oder S-Atom in der Kette enthalten kann), $C_3$-$C_7$-Cycloalkyl, $CH_2$-COO-($C_1$-$C_5$-Alkyl), Phenyl,
$R_2$ und $R_3$ gemeinsam auch $-(CH_2)_4-$,$-(CH_2)_5-$,

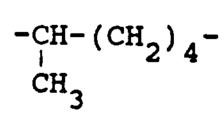

$R_4$: CN, $CONH_2$,

$R_5$ H, $CH_3$, $C_2H_5$

$R_6$ H, $CH_3$ und

X: O oder S bedeutet,

gegebenenfalls in Form von Racematen bzw. Gemischen der optischen Isomeren bzw. in Form der reinen Enantiomeren bzw. Diastereomeren, zur Bekämpfung von Piricularia an Reis

Soweit die Substituenten $R_1$ bis $R_6$ Kohlenwasserstoffketten enthalten, können diese gerade oder verzweigt und untereinander gleich oder verschieden sein. Bevorzugt sind Ketten mit bis zu 4, insbesondere bis zu 3 C-Atomen. Als Alkylsubstituent im Arylrest ist $CH_3$ bevorzugt. Halogen bedeutet Fluor, Chlor, Brom und Jod, bevorzugt Chlor und Fluor. Die Substituenten im Arylrest können gleich oder verschieden sein. $CF_3$, CN, $NO_2$, $(CH_3)_2NCH_2$, Phenyl und $C_1$-$C_5$-Alkyl-$SO_n$ sind im allgemeinen nur einmal vorhanden. Bedeutet Aryl eine Chinolinylgruppe, so handelt es sich bevorzugt um 8-Chinolinyl.

Die meisten Verbindungen der Formel I sind neu. Deshalb werden in der Erfindung Verbindungen der Formel,

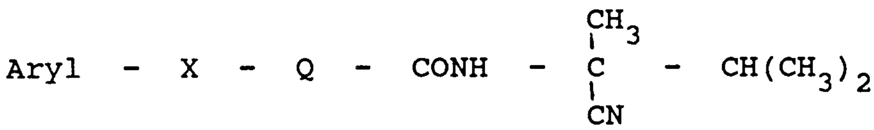

(IA)

Aryl: einen unsubstituierten oder ein- bis dreifach durch $C_1$-$C_5$-Alkyl, $C_1$-$C_5$-Alkoxy, $C_1$-$C_5$-Alkyl-$SO_n$ - (n = 0, 1 oder 2), Halogen, $NO_2$, $CF_3$, CN, $CH_3OCH_2$, $(CH_3)_2NCH_2$, COOAlkyl, $CONH_2$ oder Phenyl substituierten Phenylrest, einen 1- oder 2-Naphthyl-, einen gegebenenfalls chlorsubstituierten 2-, 3- oder 4-Pyridyl-, einen Pyrimidyl oder Chinolylrest,

Q:

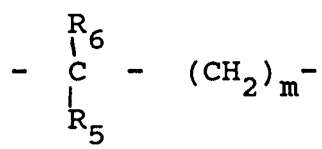

(m = 0,1 oder 2)

$R_5$: H, $CH_3$, $C_2H_5$,

$R_6$: H, $CH_3$

X: O oder S

gegebenenfalls in Form von Racematen bzw. Gemischen der optischen Isomeren bzw. in Form der reinen Enantiomeren bzw. Diastereomeren, vorgesehen.

Aryl stellt vorzugsweise 4-Chlorphenyl, 4-Chlor-2-methylphenyl, 2,4-, 3,4- oder 3,5-Dichlorphenyl dar. Es wird darüberhinaus vorgezogen, dass X O bedeutet und Q $CH(CH_3)$ bedeutet. Eine besonders bevorzugte Verbindung ist 2-(4-Chlor-2-methylphenoxy)-propionsäure-N-[2-cyano-3-methylbutyl(2)]-amid. Diese Verbindungen werden in an sich bekannter Weise hergestellt.

1. Umsetzung von Verbindungen der Formel

Aryl - X - Q - COY (II)

worin Aryl, X und Q die obige Bedeutung haben und Y eine Fluchtgruppe ("leaving group") ist, z.B. Halogen (bevorzugt Chlor), OAlkyl, OH, Acyl, mit einer Verbindung der Formel

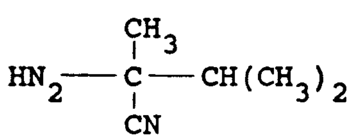

(III)

unter Abspaltung von HY.

Die Umsetzung wird vorzugsweise in einem inerten Lösungsmittel, beispielsweise Methylenchlorid, Toluol, Acetonitril, einem Ether, oder in einem Lösungsmittelgemisch bei Temperaturen zwischen Raumtemperatur und der Siedetemperatur des Reaktiongemischs durchgeführt, wobei ein HY-bindendes Mittel die Reaktion fördert, beispielsweise eine Base, wenn HY eine Säure wie HCl darstellt, Dicyclohexylcarbodiimid oder Carbonyldiimidazol, wenn HY Wasser bedeutet.

Die Ausgangsstoffe sind bekannt oder sonst nach üblichen Verfahren leicht herstellbar. So können

Verbindungen der Formel II mit Y = OH z.B. durch Umsetzung entsprechender Phenole bzw. Thiophenole Aryl-XH mit 2-Brompropionsäureethylester in Gegenwart einer Base und anschliessende Hydrolyse des Esters gewonnen werden. Aus den auf diese Weise erhaltenen Carbonsäuren entstehen, z.B. durch Umsetzung mit Thionylchlorid die entsprechenden Carbonsäurechloride der Formel II.

Die $\alpha$-Aminosäurenitrile (III), können nach der Strecker-Synthese aus dem entsprechenden Keton oder Aldehyd, NaCN und $NH_4Cl$ in Wasser bereitet werden (Houben-Weyl, Bd. VIII, S 274ff (1952).
Verbindungen der Formel III, in der die Cyangruppe durch $CONH_2$ ersetzt worden ist, entstehen aus den entsprechenden Nitrilen durch partielle Hydrolyse.

2. Umsetzung einer Verbindung der Formel

Aryl - XM (IV)

worin Aryl und X die obige Bedeutung haben und M Wasserstoff oder ein Alkalikation bedeutet, mit einer Verbindung der Formel

$$\begin{array}{c} \\ Z - Q - CONH - \end{array} \begin{array}{c} CH_3 \\ | \\ C \\ | \\ CN \end{array} \begin{array}{c} \\ - CH(CH_3)_2 \end{array} \qquad (V)$$

in der Q die obige Bedeutung hat und z Halogen oder eine Arylsulfonyloxygruppe darstellt.

Die Umsetzung erfolgt in einem inerten polaren Lösungsmittel. Wenn M = H ist, wird eine Base zugesetzt. Bevorzugt sind Bedingungen, unter denen eine Verbindung IV mit H = K oder Na gebildet wird.

Bevorzugte Bedeutung von Z ist Brom und $CH_3$-$C_6H_4$-$SO_3$-, als bevorzugtes Lösungsmittel dient Acetonitril. Die Reaktion erfolgt in der Wärme, z.B. bei Rückflusstemperatur. Als Basen können beispielsweise Alkalicarbonate, Alkalihydroxide, gegebenenfalls auch hinreichend basische Amine, etwa Triethylamin, verwendet werden.

Je nach der Bedeutung der $R_2$ bis $R_6$ können Verbindungen der Formel I mit einem oder Zwei Asymmetriezentren vorliegen. Die Isomeren können gewünschtenfalls nach üblichen Methoden getrennt oder durch Verwendung optisch aktiver Ausgangsprodukte unmittelbar synthetisiert werden.

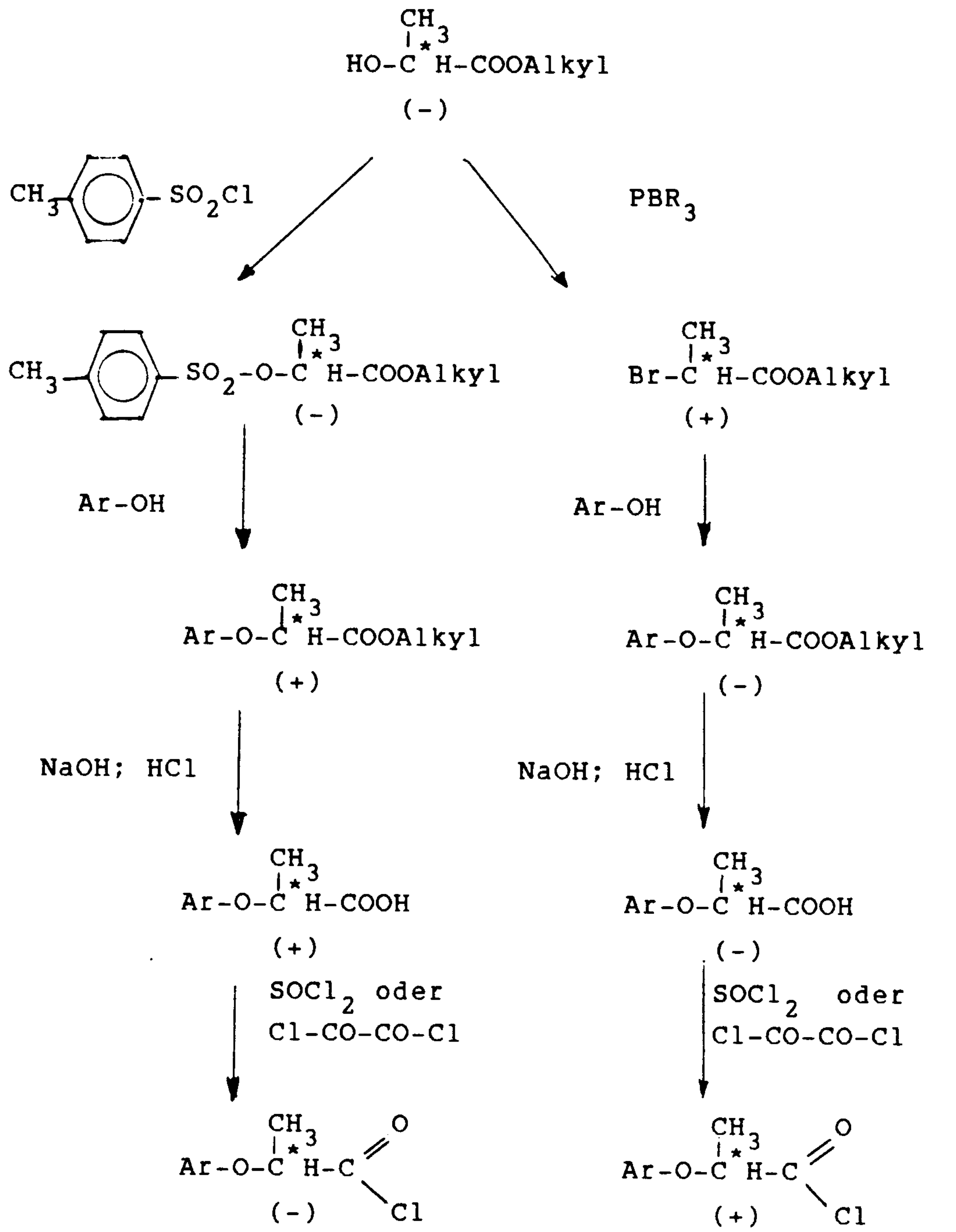


(II), (-)-Form (II), (+)-Form

"Alkyl" bedeutet vorzugsweise Methyl oder Ethyl Ähnliche Schritte führen zu optisch aktiven Ausgangsstoffen der Formel V; auch in diesem Fall können andere optisch aktive Verbindungen der Formel V entsprechend erhalten werden.

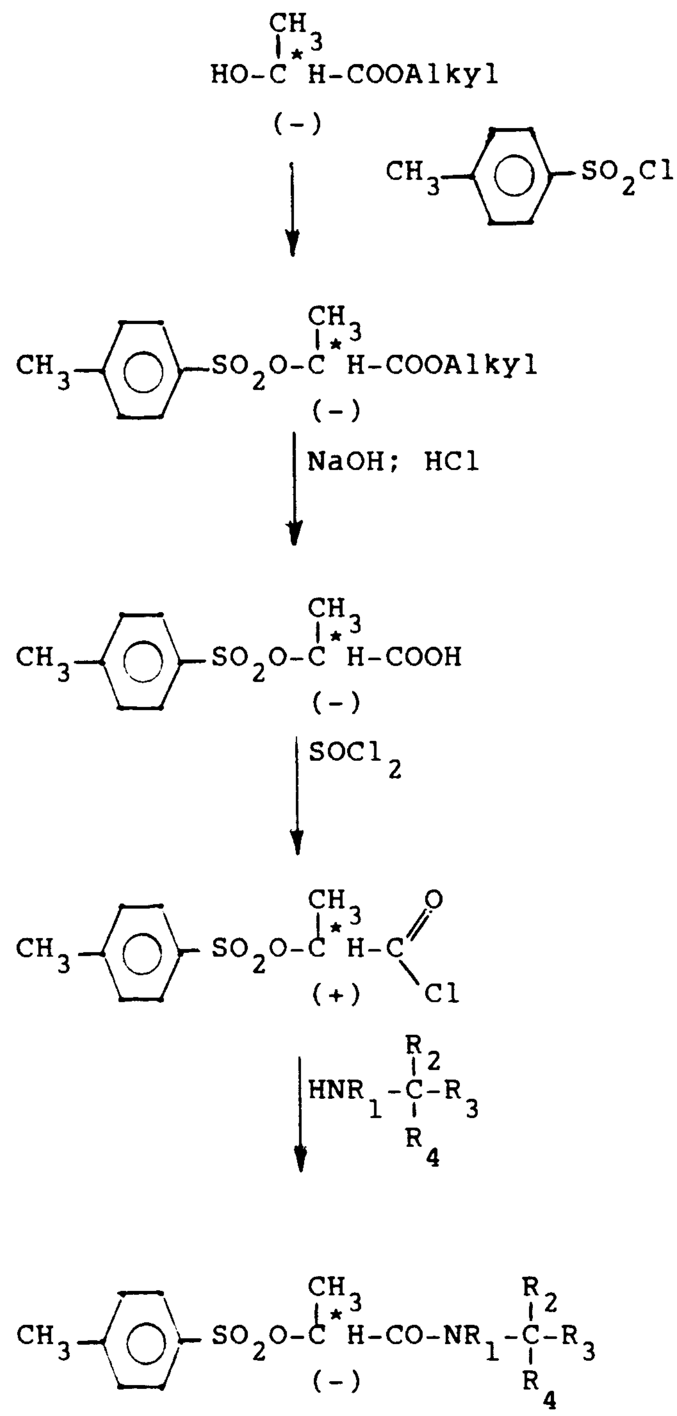


(V), (-)-Form

Die Verbindungen der Formel I wirken fungitoxisch gegen phytopathogene Pilze. Sie können insbesondere gegen Pilzkrankheiten an Reis angewandt werden. Obwohl sich die neuen Verbindungen z.T. von Herbiziden ableiten (Dichlorprop 2,4-DB), sind sie überraschenderweise gut pflanzenverträglich.

Für die Anwendung werden die Verbindungen der Formel I mit üblichen Hilfs- und/oder Trägerstoffen zu gebräuchlichen Zubereitungen verarbeitet, die für die Anwendung in Form einer Spritzbrühe mit geeigneten Mengen Wasser verdünnt werden. Solche Zubereitungen sind beispielsweise Emulsions und Lösungskonzentrate, Suspensionspulver, Stäubemittel, Granulate, die bis zu 80 Gewichtsprozent an Wirkstoff enthalten können.

Beispiele fur die Formulierung:

1. Emulsionskonzentrat

| | |
|---|---|
| 5,0 Gew.-Teile | Wirkstoff gemäss der Erfindung |
| 3,4 Gew.-Teile | epoxidiertes Pflanzenöl |
| 13,4 Gew.-Teile | eines Kombinationsemulgators aus Fettalkoholpolyglykoläther und Calcium-Alkylarylsulfonat |
| 40,0 Gew.-Teile | Dimethylformamid |
| 38,2 Gew. Teile | Xylol |

Die Komponenten werden vermischt und für die Anwendung mit Wasser auf eine Wirkstoffkonzentration von 0,01 bis 0,1 Gewichtsprozent verdünnt.

Suspensionspulver

| | |
|---|---|
| 10 Gew.-Teile | Wirkstoff gemäß der Erfindung |
| 3 Gew.-Teile | Natrium Fettalkoholsulfonat |
| 5 Gew.-Teile | Salze von Naphthalin-sulfonsäure-Formaldehydkondensat |
| 82 Gew.-Teile | Kaolin |

Die Wirkung der erfindungsgemäßen Verbindungen, z.B. gegen Piricularia wurde unter tropischen Bedingungen an Saatreis untersucht. 2 Saatreisreihen (I und II) zwischen älteren, mit Piricularia natürlich infizierten Reihen wurden am 41., 45. und 49. Tag nach der Saat mit Spritzbrühen behandelt, die bestimmte Mengen Wirkstoff enthielten. Als Vergleich diente die nur mit Wasser behandelte Kontrolle. Die Bonitierung erfolgte 6, 8, 10 und 13 Tage nach der letzten Spritzung (% befallene Pflanzen).

Die erfindungsgemäßen Verbindungen erwiesen sich als gut wirksam gegen Piricularia und als gut pflanzenverträglich.

Weitere Teste sind nachstehend beschrieben.

Wirkung gegen Piricularia an Reis

A. Blattbehandlung

Reispflanzen werden in Pikierschalen vorgezogen. Sie werden mit Emulsionen bzw. Suspensionen, die 1000, 500 oder 250 ppm des jeweiligen Wirkstoffs enthalte n, tropfnaß besprüht. Zwei Tage nach der Behandlung werden die Pikierkästen für 5-6 Tage zwischen infizierten Reispflanzen im Freiland aufgestellt, um eine Infektion herbeizuführen. Auswertung 5-8 Tage danach.

B. Bodenbehandlung

Reispflanzen werden in Blumentöpfen vorgezogen. Emulsionen bzw. Suspensionen mit 500 ppm der angegebenen Wirkstoffe werden an die Wurzeln gegossen. Zwei Tage nach der Behandlung werden die Töpfe für 4-6 Tage zwischen Reispflanzen im Freiland aufgestellt, die mit Piricularia befallen sind, um eine Infektion herbeizuführen. Die Auswertung erfolgt 5-7 Tage nach der Infektion.

Die Bonitur erfolgt mit den Noten 1 bis 3:

1: kein Befall
2: geringer Befall
3: Befall wie bei der unbehandelten Kontrolle.

Die in der Tabelle angegebenen Zahlen sind Mittelwerte aus 3 Versuchen und mehreren zeitlich abgestuften Bonituren.

C. Anwendung unter Wasser (Submerged-Anwendung).

Reispflanzen werden in mit Erde gefüllte Eimer gepflanzt. Die Erde wird flach mit Wasser überschichtet. Man gibt soviel Wirkstoff als Suspension oder Emulsion hinzu, wie einer Aufwandmenge von 8 oder 4 oder

2 kg/ha a.i. entspricht. 2 Tage nach der Behandlung werden die Testpflanzen zwischen befallenen Reispflanzen im Freiland aufgestellt und bleiben während des Versuchs der Infektion ausgesetzt. Die Auswertung beginnt einen Tag, nachdem bei der unbehandelten Kontrolle ein Befall auftritt und wird 4-5 ml durchgeführt (3 Versuche mit jeder Substanz). Bewertung wie bei A und B. Die Zahlen in der Tabelle sind Mittelwerte aus drei Versuchen und mehreren Bonituren.

## Testergebnisse

| Wirkstoff nach Beispiel | Wirkstoff-konzentration [ppm] | Befallszahl Test A | Test B | Test C kg/ha | Befalls- |
|---|---|---|---|---|---|
| Tab. II Nr. 62 | 1000 | 1,0 | | | |
| | 500 | 1,0 | 1,8 | 8 | 2,0 |
| | 250 | 1,0 | | | |
| Beispiel Nr. 9 | 1000 | 1,0 | | 4 | 1,8 |
| | 500 | 1,0 | 2,0 | 2 | 2,2 |
| | 250 | 1,5 | | | |
| Tab. II Nr. 1 | 1000 | - | | | |
| | 500 | 1,0 | 2,5 | 8 | 1,8 |
| | 250 | 1,0 | | | |
| Tab. V Nr. 2 | 1000 | 1,0 | | | |
| | 500 | 1,5 | 1,0 | 4 | 1,5 |
| | 250 | 2,5 | | | |
| Beispiel Nr. 4 | 1000 | 1,0 | | 8 | 1,8 |
| | 500 | 1,8 | 2,1 | | |
| | 250 | 2,1 | | | |
| Tab. II Nr. 2 | 1000 | 1,1 | | | |
| | 500 | 1,0 | - | 4 | 2,0 |
| | 250 | 2,0 | | | |
| Tab. II Nr. 33 | 1000 | 1,0 | | | |
| | 500 | 1,0 | - | | |
| | 250 | 2,0 | | | |
| Beispiel Nr. 6e | 1000 | 1,5 | | | |
| | 500 | 2,0 | 2,0 | | |
| | 250 | 2,0 | | | |
| Tab. IV Nr. 6 | 1000 | - | | | |
| | 500 | 1,0 | 1,9 | | |
| | 250 | 1,0 | | | |

Beispiel 1

2-(2,4-Dichlorphenoxy)-propionsäure-N-(1-ethyl-1-cyanopropyl)-amid

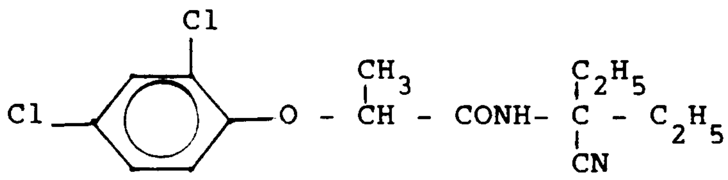

2,2 g 3-Amino-3-cyanopentan und 2,4 g Triethylamin werden in 100 ml Methylenchlorid gelöst. Hierzu gibt man 5,1 g 2-(2,4-Dichlorphenoxy)-propionsäurechlorid und läßt den Ansatz über Nacht bei Raumtemperatur rühren. Die Lösung wird mit Wasser und Natriumhydrogencarbonatlösung ausgeschüttelt, getrocknet und eingeengt. Als Rückstand erhält man 6,3 g (96 % d.Th.) eines bräunlichen, viskosen Öls, das beim Verrühren mit Diisopropylether kristallisiert.
Ausbeute: 4,9 g weißer Feststoff (74 % d.Th.)
Schmelzpunkt: 100 - 102 °C.

Die Struktur wird durch die spektroskopische Untersuchung bestätigt.

| Analyse: $C_{15}H_{18}Cl_2N_2O_2$ M = 329,23 | | | | |
|---|---|---|---|---|
| | C % | H % | Cl % | N % |
| gef.: | 54,58 | 5,54 | 21.06 | 8,35 |
| ber.: | 54,72 | 5,51 | 21,54 | 8,51 |

Beispiel 2

2-(4-Methylthiophenyloxy)-propionsäure-N-(1-cyano-1,2-dimethylpropyl)-amid

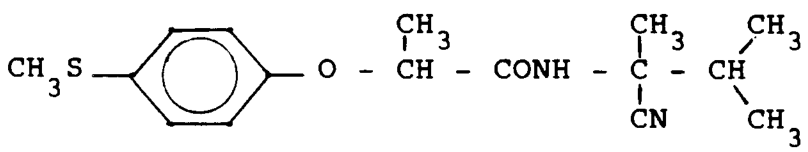

2,5 g 2-Brompropionsäure-N-(1-cyano-1,2-dimethylpropyl)-amid (hergestellt analog Beispiel 5a) und 1,4 g 4-Methylmercaptophenol werden in 50 ml Methylisobutylketon gelöst. Nach Zugabe von 1,5 g Pottasche wird der Ansatz 3 Stunden bei 80 °C gerührt. Die Lösung wird abgesaugt und eingeengt. Man erhält 2,8 g bräunliches Öl (91,5 %), das beim Verrühren mit Diisopropylether kristallisiert.
Fp. 83-86 °C

Analyse: $C_{16}H_{22}N_2O_2S$ M = 306,43

| | C % | H % | N % | S % |
|---|---|---|---|---|
| gef.: | 62,48 | 7,24 | 9,23 | 10,34 |
| ber.: | 62,71 | 7,24 | 9,14 | 10,46 |

Die Struktur wurde spektroskopisch bestätigt.

Beispiel 3

2-(4-Chlor-2-methylphenoxy)-priopionsäure-N-[3-cyanopentyl(3)-]-amid

4,4 g 2-Amino-2-ethylbutyronitril (0,039 mol) und 4,6 g Triethylamin (0,046 mol) werden in Methylenchlorid gelöst, 9,0 g
2-(4-Chlor-2-methylphenoxy)-propionsäurechlorid (0,039 mol) werden unter Rühren zugetropft. Der Ansatz erwärmt sich. Er wird 3 Stunden ohne Heizung nachgerührt, nacheinander mit Wasser und Bicarbonatlösung ausgeschüttelt, getrocknet und eingeengt. Als Rückstand bleibt ein braunes Öl (10,8 g), das nach Verrühren mit Isopropyläther kristallisiert. Das Produkt wird abgesaugt und getrocknet.
Ausbeute: 10,6 g (88 % d.Th.) weißer Feststoff
Fp.: 125 - 126 ° C.

Elementaranalyse und NMR-Spektrum bestätigen die angegebene Formel.

Beispiel 4

2-(4-Chlor-2-methylphenoxy)-propionsäure-N-[2-cyano-3-methylbutyl(2)-]-amid

Analog Beispiel 3 wird die Titelverbindung aus äquimolaren Mengen 2-Amino-2,3-dimethylbutyronitril erhalten.
Ausbeute: 76 % d.Th.; Fp.: 97 - 99 ° C.

Das Produkt fällt zunächst als braunes Öl an. Es besteht aus 4 Isomeren. Das Gemisch kann durch stufenweises Ausfällen mit kaltem Äther in 3 Fraktionen zerlegt werden.

Aus 11,1 g Öl erhält man:

Fraktion I: 1,8 g weißer Feststoff,
Fp.: 117-118 ° C
Fraktion II: 1,8 g bräunlicher Feststoff,
Fp.: 94-96 ° C;
Fraktion III: 5,6 g rötliches Öl (chromatographisch gereinigt)

Die NMR-Spektroskopie zeigt eine Enantiomerenpaaranreicherung in den Fraktionen I und II:

Fraktion I Enantiomerenpaar I zu Enantiomerenpaar II
89 : 11 (Diastereomerenverhältnis)
Fraktion II Enantiomerenpaar I zu Enantiomerenpaar II
26 : 74

Durch Umkristallisieren der Fraktionen können die Enentiomerenpaare weiter angereichert werden.

Ensprechend den vorstehenden Beispielen werden auch die folgenden Verbindungen der folgenden Formel erhalten:

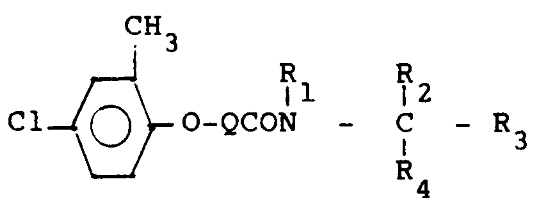

## Tabelle I

| Nr. | Q | $R_2$ | $R_3$ | $R_1$ | $R_4$ | Fp. [°C] |
|---|---|---|---|---|---|---|
| 1 | $CH_2$ | $C_2H_5$ | $C_2H_5$ | H | CN | 86-88 |
| 2 | $CH(CH_3)$ | $i-C_3H_7$ | $CH_3$ | H | $CONH_2$ | 108-111 |
| 3 | $CH_2$ | $i-C_3H_7$ | $CH_3$ | H | $CONH_2$ | 105-107 |
| 4 | $CH_2$ | $i-C_3H_7$ | $CH_3$ | H | CN | 102-103 |
| 5 | $CH(CH_3)$ | $n-C_3H_7$ | $CH_3$ | H | CN | 71-75 |
| 6 | $CH(CH_3)$ | $C_2H_5$ | $CH_3$ | H | CN | 86-87 |
| 7 | $CH(CH_3)$ | $n-C_3H_7$ | $CH_3$ | H | $CONH_2$ | 100-102 |
| 8 | $CH(CH_3)$ | $n-C_5H_{11}$ | $CH_3$ | H | CN | |
| 9 | $CH(CH_3)$ | $-(CH_2)_5-$ | | H | CN | 134-136 |
| 10 | $CH_2$ | $-(CH_2)_5$ | | H | CN | 118-120 |
| 11 | $CH_2$ | $i-C_3H_7$ | $CH_3$ | $CH_3$ | CN | |
| 12 | $CH(CH_3)$ | $i-C_3H_7$ | $CH_3$ | $CH_3$ | CN | Öl |
| 13 | $CH(CH_3$ | $-(CH_2)_4-$ | | H | CN | 143-146 |
| 14 | $CH(CH_3)$ | $-\underset{CH_3}{\underset{\vert}{CH}}-(CH_2)_4$ | | H | CN | 121-127 |

Beispiel 5

2-(4-Chlorphenylthio)-propionsäure-N-(1-ethyl-1-cyanopropyl)-amid

a) 2-Brompropionsäure-N-(1-ethyl-1-cyanopropyl)-amid

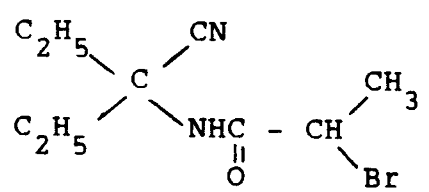

Zu 88,4 g 2-Bromopropionsäureanhydrid (0,325 mol), in 280 ml Methylenchlorid gelöst, werden unter Kühlen im Eisbad 36,5 g 2-Amino-2-cyano-n-pentan in 100 ml Methylenchlorid während 40 Minuten unter Rühren zugetropft. Nach Rühren über Nacht wird die Lösung mit Wasser und Natriumbicarbonatlösung ausgeschüttelt, getrocknet und eingeengt. Das zurückbleibende Öl wird mit wenig Ether verrieben, wobei das Produkt auskristallisiert.

Fp. 85-87 ° C
Ausbeute: 61,2 g (76,5 % d.Th.)
b)

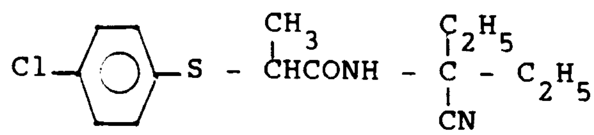

5,8 g 4-Chlorthiophenol (0,04 mol) werden in 150 ml Methylisobutylketon mit 12,2 g Kaliumcarbonat 10 Minuten bei 90 ° C verrührt. Zu der entstandenen Suspension gibt man unter Rühren 9,9 g 2-Brompropionsäure-N-(1-ethyl-1-cyanopropyl)-amid und läßt den Ansatz ca. 5 Stunden bei ca. 90 ° C rühren. Die Lösung wird filtriert, nacheinander mit Wasser, 2N Natronlauge und Wasser ausgeschüttelt, mit Magnesiumsulfat getrocknet und eingeengt. Man erhält ein braunes Öl, das beim Verrühren mit wenig Ether zu einer bräunlichen Kristallmasse erstarrt.
Fp. 108-110 ° C
Ausbeute: 7,2 g (58,1 % d.Th.)

Elementaranalyse:

| | C | H | B | Cl | S |
|---|---|---|---|---|---|
| ber.: | 57,96 % | 6,16 % | 9,01 % | 11,4 % | 10,32 % |
| gef.: | 57,77 % | 6,35 % | 8.86 % | 11.32 % | 10,28 % |

Entsprechend den vorstehendem Beispiel erhält man auch folgende Verbindung

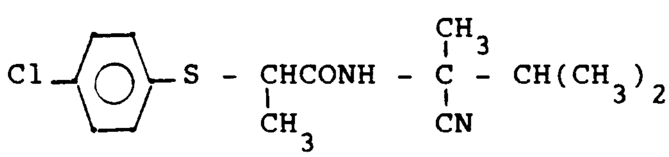

Fp. 106-109 ° C.

Beispiel 6

(+)-2-(4-Chlor-2-methylphenoxy)-propionsäure-N-(1-ethyl-1-cyanopropyl)-amid

a) (-)-0-(4-methylphenylsulfonyl)-milchsäuremethylester
Zu einer Lösung von 25,2 g S-(-)-Milchsäuremethylester und 46,1 g p-Toluolsulfonsäurechlorid in 160 ml Toluol werden 26,9 g Triethylamin zugetropft. Man läßt über Nacht nachrühren und saugt vom Niederschlag ab. Die Toluollösung wird mit verdünnter Salzsäure und Wasser ausgeschüttelt, mit Natriumsulfat getrocknet und eingeengt. Man erhält 55,9 g farbloses Öl, das durch Vakuumdestillation gereinigt wird.
$Kp_{0,2}$: 148-152 ° C $[\alpha]_D^{24}$ : -50,1 ° (Ethanol)
Ausbeute: 43,5 g (70 % d.Th.)
b) (+)-(4-Chlor-2-methylphenoxy)-propionsäuremethyl- ester
41,9 g S-(-)-O-(4-Methylsulfonyl)-milchsäuremethyl-ester und 23,1 g 4-Chlor-2-methylphenol werden in 100 ml Acetonitril gelöst, mit 50 g Pottasche versetzt und 10 Stunden unter Rückfluß gerührt. Die Lösung wird abgesaugt und eingeengt. Der Rückstand wird in Toluol aufgenommen, mit 1N Natronlauge ausgeschüttelt, getrocknet und eingeengt. Man erhält 32,3 g rötliche Flüssigkeit (87 % d.Th.)
c) (+)-4-Chlor-2-methylphenoxypropionsäure
Das nach b) hergestellte Rohprodukt (32,2 g) wird in 100 ml Aceton gelöst. Unter Rühren und Eiskühlung

wird eine Lösung von 6,8 g NaOH in 30 ml Wasser zugetropft. Nach Rühren über Nacht wird der Ansatz mit Wasser verdünnt und mit Methyenchlorid ausgeschüttelt. Die wässrige Lösung wird mit konz. Salzsäure angesäuert und das ausgeschiedene Produkt mit Methylenchlorid ausgeschüttelt. Die Methylenchloridlösung wird abgetrennt, getrocknet und eingeengt. Man erhält einen öligen Rückstand, der sofort erstarrt.
Fp. 62-72 ° C (auf Ton abgepreßt)
$[\alpha]_D^{24}$ : + 14,1 ° (Ethanol)
Ausbeute: 27,7 g (91 % d.Th.)
d) (-)-4-Chlor-2-methylphenoxypropionsäurechlorid
27,2 g (+)-4-Chlor-2-methylphenoxypropionsäure und 30,2 g Thionylchlorid werden mit 100 ml Toluol 3 Stunden bei 100 ° C gerührt. Die Lösung wird im Vakuum eingeengt. Man erhält 29,6 g braunes Öl, das ohne Reinigung umgesetzt wird.
e) (+)-2-(4-Chlor-2-methylphenoxy)-propionsäure-N-(1-ethyl-1-cyanopropyl)-amid
8,4 g des Rohprodukts aus d) werden zu 4 g 3-Amino-3-cyano-n-pentan und 4,4 g Triethylamin, gelöst in 100 ml Toluol, bei -20 ° bis -30 ° C unter Rühren zugetropft. Anschließend wird der Ansatz bei RT 3 Stunden nachgerührt, mit Wasser ausgeschüttelt und die Lösung eingeengt. Der ölige Rückstand (8,8 g) wird Produkt ausfällt, das abgetrennt wird.
Ausbeute 2,8 g (25 % d.Th.)
Fp: 98 - 100 ° C
$[\alpha]_D^{22}$ : + 9,1 ° (Ethanol)

## Beispiel 7

(-)-2-(4-Chlor-2-methylyphenoxy)-propionsäure-N-(1-ethyl-1-cyanopropyl)-amid

a) (+)-2-Brompropionsäureethylester
47,2 g S-(-)-Milschsäureethylester werden in 300 ml Methylenchlorid gelöst. 108 g Phosphortribromid werden zugetropft. Die Reaktion ist exotherm. Nach Rühren über nacht bei RT wird der Ansatz auf Eis gegossen und mit Wasser ausgerührt. Die Methylenchloridlösung wird mit Bicarbonatlösung ausgeschüttelt, getrocknet und eingeengt. Der Rückstand wird destilliert.
Ausbeute: 33,8 g; farbloses Öl (47 % d.Th.)
$Kp_{25\ mbar}$ 55-56 ° c.
b) (-)-(4-Chlor-2-methylphenoxy)-propionsäureethyl- ester
Das unter a) beschriebene Produkt (33,8 g) wird zusammen mit 26,7 g 4-Chlor-2-methylphenol in 300 ml Toluol gelöst und nach Zugabe von 52 g $K_2CO_3$ 10 Stunden unter Rückfluß gerührt. Die Lösung wird abgesaugt, 2 x mit 1N Natronlauge ausgeschüttelt, getrocknet und eingeengt. Man erhält 34,3 g klare Flüssigkeit (76 % d.Th.)
$[\alpha]_D^{22}$ : -14,46 ° (Ethanol)
c) (-)-(4-Chlor-2-methylphenoxy)-propionsäure (-)-CMPP)
Die Hydrolyse des nach b) erhaltenen Esters erfolgt analog Beispiel 6c).
Aus 24,2 g
(-)-(4-Chlor-2-methylphenoxy)-propionsäureethylester erhält man 19,7 g (-)-(4-Chlor-2-methylphenoxy)-propionsäure (92 % d.Th.).
Fp.: 69-75 ° C
$[\alpha]_D^{22}$ : -9,679 ° (Ethanol)
d) (+)-(4-Chlor-2-methylphenoxy)-propionsäurechlorid ((+)-CMPP-chlorid)
Die unter 7c) beschriebene Säure wird analog Beispiel 6d) in das Säurechlorid übergeführt, welches ohne Reinigung weiterverarbeitet wird.
Aus 8,6 g (-)-CMPP erhält man 8,4 g (+)-CMPP-chlorid als bräunliches Öl (90 % d.Th.)
$[\alpha]_D^{22}$ : + 4,486 ° ($CCl_4$
e) (-)-2-(4-Chlor-2-methylphenoxy)-propionsäure-N-(1-ethyl-1-cyanopropyl)-amid
Das (+)-CMPP-chlorid wird wie in Beispiel 6e) beschrieben mit 3-Amino-3-cyano-n-pentan umgesetzt. Man erhält aus 8 g (+)-CMPP-chlorid 3 g (-)-2-(4-Chlor-2-methylphenoxy)-propionsäure-N-(1-ethyl-1-cyanopropyl)-mid (28 % d.Th.)
Fp. 98-100 ° C
$[\alpha]_D^{22}$ : - 8,584 ° (Ethanol)
Eine Variante zur Herstellung der rechtsdrehenden Phenoxypropionsäureamide wird nachstehend am Beispiel des (+)-2-(4-Chlor-2-methylphenoxy)-propionsäure-N-(1-ethyyl-1-cyanopropyl)-amids dargestellt:

Beispiel 8

(+)-2-(4-Chlor-2-methylphenoxy)-propionsäure-N-(1-ethyl-1-cyanopropyl)-amid

a) (+)-O-(4-Methylphenylsulfonyl)-milchsäurechlorid
17,9 g (-)-O-(4-Methylphenylsulfonyl)-milchsäure (Helv. Chim. Acta 65/1240 (1982)) und 13 g Thionylchlorid werden 3 Stunden bei 95-100°C gerührt. Das Produkt wird im Vakuum eingeengt und entgast. Man erhält 19,2 g braunes Öl (100 % d.Th.).
b) (-)-O-(4-Methylsulfonyl)-milchsäure-N-(1-ethyl-1-cyanopropyl)-amid
18,8 g des Rohprodukts aus 8a) werden bei -20 bis -30°C zu einer Lösung von 8 g 3-Amino-3-cyano-n-pentan und 8,8 g Triethylamin in 200 ml Toluol getropft. Der Ansatz wird bei -20°C 3 Stunden gerührt und anschließend über Nacht bei RT. Die Lösung wird mit Wasser ausgeschüttelt und eingeengt. Man erhält 23,4 g braunes, klares Öl (96 % d.Th.), das durch Verrühren mit Diisopropylether kristallisiert wird.
Fp. 57-60°C $[\alpha]_D^{22}$ - 40,4° (Ethanol)
Ausbeute: 8,4 g (35 % d.Th.)
c) (+)-2-(4-Chlor-2-methylphenoxy)-propionsäure-N-(1-ethyl-1-cyanopropyl)-amid
4,7 g (-)-O-(4-Methylsulfonyl)-milchsäure-N-(1-ethyl-1-cyanopropyl)-amid und 2 g 4-Chlor-2-methylphenol werden in 100 ml Toluol gelöst. Man gibt 4,5 g gepulverte Pottasche hinzu und erhitzt den Ansatz 12 Stunden zum Rückfluß unter Rühren. Die Lösung wird abgesaugt, mit 1N Natronlauge ausgeschüttelt und eingeengt. Man erhält 3,1 g gelbes Öl (72 % d.Th.), das beim Verrühren mit Diisopropylether kristallisiert. Man erhält 2,2 g weißen kristallinen Feststoff (51 % d.Th.)..
Fp. 97-99°C $[\alpha]_D^{22}$ : + 11,94° (Ethanol)

Entsprechend den Beispielen 6 bis 8 werden auch rechts-und linksdrehende Enantiomeren der folgenden Verbindung hergestellt:

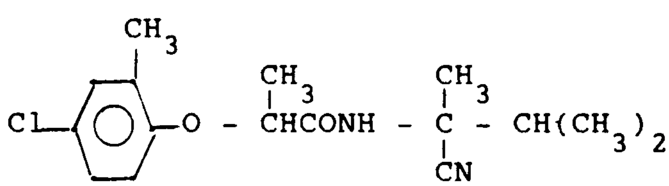

Beispiel 9: $[\alpha]_D^{22}$ = + 9,1° (Ethanol)

Beispiel 10: $[\alpha]_D^{22}$ = - 7,65° (Ethanol)

Öle, Diastereomerengemische

TABELLE II

Verbindungen der Formel

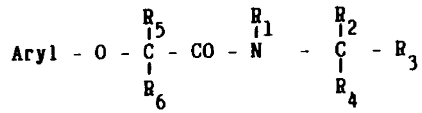

| Nr. | Aryl | $R_1$ | $R_2$ | $R_3$ | $R_4$ | $R_5$ | $R_6$ | Fp (°C) |
|---|---|---|---|---|---|---|---|---|
| 1 | 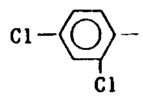 | H | $CH_3$ | $CH(CH_3)_2$ | CN | H | $CH_3$ | 79-80 |
| 2 | " | H | $CH_3$ | $C_2H_5$ | CN | H | $CH_3$ | 105-107 |
| 3 | " | $CH_3$ | $CH_3$ | $CH(CH_3)_2$ | CN | H | $CH_3$ | Öl |
| 4 | " | H | $-(CH_2)_5-$ | | CN | H | $CH_3$ | 142-144 |
| 5 | " | H | $CH_3$ | $n-C_5H_{11}$ | CN | H | $CH_3$ | 65-67 |
| 6 | " | H | $CH_3$ | $CH_2CH_2CH(CH_3)_2$ | CN | H | $CH_3$ | 74-76 |

| Nr. | Aryl | $R_1$ | $R_2$ | $R_3$ | $R_4$ | $R_5$ | $R_6$ | Fp (°C) |
|---|---|---|---|---|---|---|---|---|
| 7 | 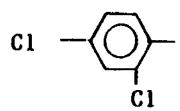 | H | $C_2H_5$- | $CH_2CH(CH_3)_2$ | CN | H | $CH_3$ | 70-75 |
| 8 | " | H | $CH_3$ | $CH_2CH(CH_3)_2$ | CN | H | $CH_3$ | 74-75 |
| 9 | " | H | $-(CH_2)_4$ | | CN | H | $CH_3$ | 117-119 |
| 10 | " | H | H | $CH(CH_3)_2$ | CN | H | $CH_3$ | Öl |
| 11 | " | H | $CH_3$ | $-CH_2OCH_3$ | CN | H | $CH_3$ | Öl |
| 12 | " | H | $-CH(CH_3)-(CH_2)_4$ | | CN | H | $CH_3$ | 129-134 |
| 13 | " | H | $CH_3$ | $-CH_2COOC_2H_5$ | CN | H | $CH_3$ | |

| Nr. | Aryl | $R_1$ | $R_2$ | | $R_3$ | $R_4$ | $R_5$ | $R_6$ | Fp (°C) |
|---|---|---|---|---|---|---|---|---|---|
| 14 | 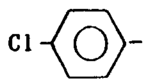 | H | $CH_3$ | | $CH(CH_3)_2$ | CN | H | H | 85-87 |
| 15 | | H | | $-(CH_2)_5-$ | | CN | H | H | 94-96 |
| 16 | " | H | $C_2H_5$ | | $C_2H_5$ | CN | H | H | 50-52 |
| 17 | " | H | $CH_3$ | | $CH(CH_3)_2$ | CN | H | $CH_3$ | 80-81 |
| 18 | " | $CH_3$ | $CH_3$ | | $CH(CH_3)_2$ | CN | H | $CH_3$ | Öl |
| 19 | " | H | $C_2H_5$ | | $C_2H_5$ | CN | H | $CH_3$ | 83-84 |
| 20 | " | H | $CH_3$ | | $n-C_5H_{11}$ | CN | H | $CH_3$ | 75-77 |
| 21 | " | H | $CH_3$ | | $CH_2CH(CH_3)_2$ | CN | H | $CH_3$ | 58-62 |

| Nr. | Aryl | $R_1$ | $R_2$ | $R_3$ | $R_4$ | $R_5$ | $R_6$ | Fp (°C) |
|---|---|---|---|---|---|---|---|---|
| 22 | $C_6H_5$ | H | $CH_3$ | $CH(CH_3)_2$ | CN | H | $CH_3$ | 81-83 |
| 23 | 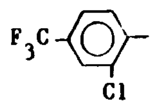 | H | $CH_3$ | $CH(CH_3)_2$ | CN | H | $CH_3$ | 120-124 |
| 24 | 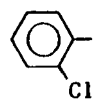 | H | $CH_3$ | $CH(CH_3)_2$ | CN | H | $CH_3$ | 60-62 |
| 25 | " | H | $CH_3$ | $n-C_5H_{11}$ | CN | H | $CH_3$ | 97-100 |
| 26 | 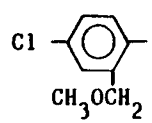 | H | $CH_3$ | $CH(CH_3)_2$ | CN | H | $CH_3$ | Öl |
| 27 | 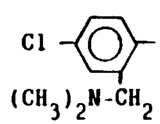 | H | $CH_3$ | $CH(CH_3)_2$ | CN | H | $CH_3$ | Öl |
| 28 | 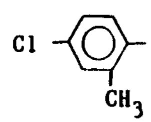 | H | $CH_3$ | $CH(CH_3)_2$ | CN | H | $C_2H_5$ | 98-100 |

| Nr. | Aryl | $R_1$ | $R_2$ | $R_3$ | $R_4$ | $R_5$ | $R_6$ | Fp (°C) |
|---|---|---|---|---|---|---|---|---|
| 29 | 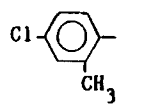 | H | $CH_3$ | $CH(CH_3)_2$ | CN | $CH_3$ | $CH_3$ | 94-97 |
| 30 | 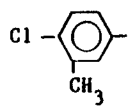 | H | $CH_3$ | $CH(CH_3)_2$ | CN | H | $CH_3$ | 110 |
| 31 | " | H | $CH_3$ | $n-C_5H_{11}$ | CN | H | $CH_3$ | Öl |
| 32 | " | H | $CH_3$ | $CH(CH_3)_3$ | $CONH_2$ | H | $CH_3$ | 155 |
| 33 | 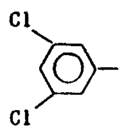 | H | $CH_3$ | $CH(CH_3)_3$ | CN | H | $CH_3$ | 105 |
| 34 | " | H | $CH_3$ | $CH(CH_3)_3$ | $CONH_2$ | H | $CH_3$ | 140 |

| Nr. | Aryl | $R_1$ | $R_2$ | $R_3$ | $R_4$ | $R_5$ | $R_6$ | Fp (°C) |
|---|---|---|---|---|---|---|---|---|
| 35 | 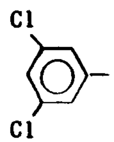 | H | $CH_3$ | $n-C_5H_{11}$ | CN | H | $CH_3$ | Öl |
| 36 | 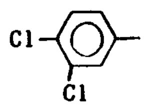 | H | $CH_3$ | $CH(CH_3)_2$ | CN | H | $CH_3$ | 103 |
| 37 | " | H | $CH_3$ | $CH(CH_3)_2$ | $CONH_2$ | H | $CH_3$ | 150 |
| 38 | " | H | $CH_3$ | $n-C_5H_{11}$ | CN | H | $CH_3$ | Öl |
| 39 | 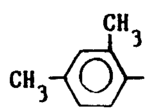 | H | $CH_3$ | $CH(CH_3)_2$ | CN | H | $CH_3$ | 106 |
| 40 | " | H | $CH_3$ | $n-C_3H_7$ | CN | H | $CH_3$ | 142 |

| Nr. | Aryl | $R_1$ | $R_2$ | $R_3$ | $R_4$ | $R_5$ | $R_6$ | Fp (°C) |
|---|---|---|---|---|---|---|---|---|
| 41 | 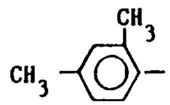 | H | $C_2H_5$ | $C_2H_5$ | CN | H | $CH_3$ | 114 |
| 42 | " | H | $-(CH_2)_5$ | | CN | H | $CH_3$ | 121 |
| 43 | " | $CH_3$ | $CH_3$ | $CH(CH_3)_2$ | $CONH_2$ | H | $CH_3$ | 85-90 |
| 44 | 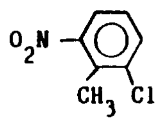 | H | $CH_3$ | $CH(CH_3)_2$ | CN | H | $CH_3$ | 160 |
| 45 | " | H | $C_2H_5$ | $C_2H_5$ | CN | H | $CH_3$ | 152-154 |
| 46 | 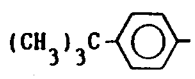 | H | $CH_3$ | $CH(CH_3)_2$ | CN | H | $CH_3$ | 88-90 |
| 47 | 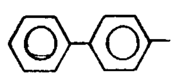 | H | $CH_3$ | $CH(CH_3)_2$ | CN | H | $CH_3$ | 106-108 |

| Nr. | Aryl | $R_1$ | $R_2$ | $R_3$ | $R_4$ | $R_5$ | $R_6$ | Fp (°C) |
|---|---|---|---|---|---|---|---|---|
| 48 | 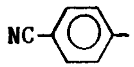 | H | $CH_3$ | $CH(CH_3)_2$ | CN | H | $CH_3$ | 98-100 |
| 49 | 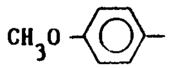 | H | $CH_3$ | $CH(CH_3)_2$ | CN | H | $CH_3$ | 57-63 |
| 50 | 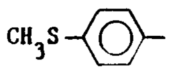 | H | $C_2H_5$ | $C_2H_5$ | CN | H | $CH_3$ | |
| 51 | 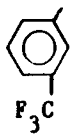 | H | $CH_3$ | $CH(CH_3)_2$ | CN | H | $CH_3$ | 78-81 |
| 52 | 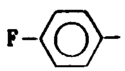 | H | $CH_3$ | $CH(CH_3)_2$ | CN | H | $CH_3$ | 72-75 |
| 53 | 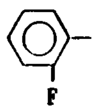 | H | $CH_3$ | $CH(CH_3)_2$ | CN | H | $CH_3$ | 98-102 |
| 54 | 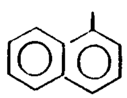 | H | $CH_3$ | $CH(CH_3)_2$ | CN | H | $CH_3$ | 152-157 |

| Nr. | Aryl | $R_1$ | $R_2$ | $R_3$ | $R_4$ | $R_5$ | $R_6$ | Fp (°C) |
|---|---|---|---|---|---|---|---|---|
| 55 | 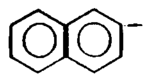 | H | $CH_3$ | $CH(CH_3)_2$ | CN | H | $CH_3$ | 90-95 |
| 56 | 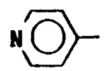 | H | $CH_3$ | $CH(CH_3)_2$ | CN | H | $CH_3$ | |
| 57 | 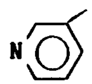 | H | $CH_3$ | $CH(CH_3)_2$ | CN | H | $CH_3$ | 80 |
| 58 | 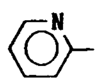 | H | $CH_3$ | $CH(CH_3)_2$ | CN | H | $CH_3$ | 125-128 |
| 59 | 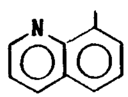 | H | $CH_3$ | $CH(CH_3)_2$ | CN | H | $CH_3$ | 105-107 |
| 60 | " | H | $CH_3$ | $CH_2CH(CH_3)_2$ | CN | H | $CH_3$ | |
| 61 | 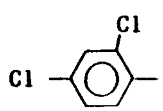 | H | $CH(CH_3)_2$ | $CH(CH_3)_2$ | CN | H | $CH_3$ | 82-84 |

| Nr. | Aryl | $R_1$ | $R_2$ | $R_3$ | $R_4$ | $R_5$ | $R_6$ | Fp (°C) |
|---|---|---|---|---|---|---|---|---|
| 62 | 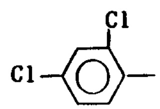 | H | $CH_3$ | 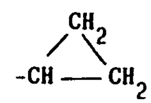 | CN | H | $CH_3$ | 89-94 |
| 63 | " | H | $CH_3$ | 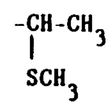 | CN | H | $CH_3$ | 124-128 |
| 64 | " | H | $CH_3$ | $CH_2-COOC_2H_5$ | CN | H | $CH_3$ | |
| 65 | " | H | $CH_3$ | 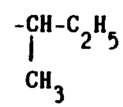 | CN | H | $CH_3$ | 107-110 |
| 66 | 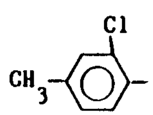 | H | $CH_3$ | $CH(CH_3)_2$ | CN | H | $CH_3$ | |

TABELLE III

Verbindungen der Formel

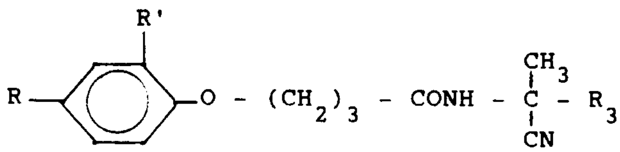

| Nr. | $R_3$ | R | R' | Fp.(°C) |
|---|---|---|---|---|
| 1 | $CH(CH_3)_2$ | Cl | $CH_3$ | Öl |
| 2 | $n\text{-}C_5H_{11}$ | Cl | $CH_3$ | Öl |
| 3 | $CH(CH_3)_2$ | $CH_3$ | Cl | |
| 4 | $n\text{-}C_5H_{11}$ | $CH_3$ | Cl | |

Tabelle IV

Verbindungen der Formel

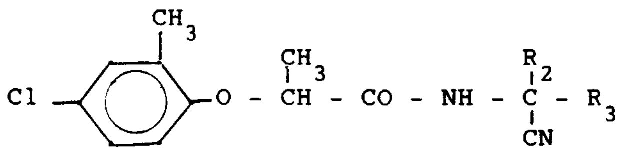

| Nr. | $R_2$ | $R_3$ | Fp.[°C] |
|---|---|---|---|
| 1 | $CH_3$ | $n-C_3H_7$ | 71-75 |
| 2 | $CH_3$ | $C_2H_5$ | 86-87 |
| 3 | $C_2H_5$ | $C_2H_5$ | 125-126 |
| 4 | $CH_3$ | $CH_2CH(CH_3)_2$ | 101-102 |
| 5 | $CH_3$ | $CH_2OCH_3$ | Öl |
| 6 | H | $CH(CH_3)_2$ | 86-88 |
| 7 | $CH_3$ | $CH_2CH_2CH(CH_3)_2$ | 92-94 |
| 8 | $C_2H_5$ | $CH_2CH(CH_3)_2$ | 68-70 |
| 9 | $CH_3$ | $CH(CH_3)C_2H_5$ | 103-109 |
| 10 | $CH(CH_3)_2$ | $CH(CH_3)_2$ | 106-109 |
| 11 | $CH_3$ | 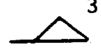 | 87-88 |
| 12 | $CH_3$ | $-CH(SCH_3)-CH_3$ | 127-129 |
| 13 | $CH_3$ | $CH_3$ | 128-130 |
| 14 | $CH_3$ | $C(CH_3)_3$ | 110-115 |
| 15 | H | $C_2H_5$ | 64-66 |
| 16 | H | $CH_3CH_2CH_3$ | 66-70 |
| 17 | $CH_3$ | $C_6H_5$ | 148-152 |
| 18 | $CH_3$ | $CH_2SC_2H_5$ | 78-82 |

Tabelle V

Verbindungen der Formel

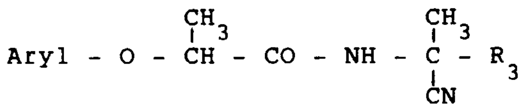

| Nr. | Aryl | $R_3$ | Fp.[°C] |
|---|---|---|---|
| 1 | 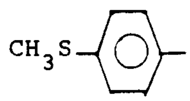 | $CH(CH_3)_2$ | 83-86 |
| 2 | 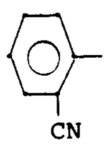 | $CH(CH_3)_2$ | 74-76 |
| 3 | 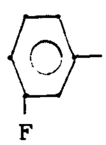 | $CH(CH_3)_2$ | 71-75 |
| 4 | 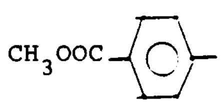 | $CH(CH_3)_2$ | 110-115 |
| 5 | 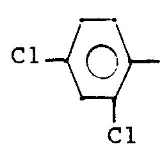 | $CH_3$ | 134-137 |

| Nr. | Aryl | $R_3$ | Fp. [°C] |
|---|---|---|---|
| 6 | 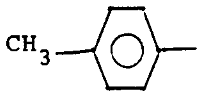 | $CH(CH_3)_2$ | 80-82 |
| 7 | 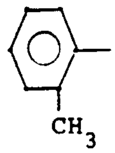 | $CH(CH_3)_2$ | 74-76 |
| 8 | 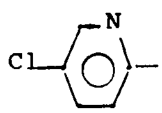 | $CH(CH_3)_2$ | 134-136 |
| 9 | 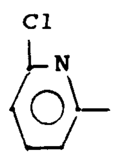 | $CH(CH_3)_2$ | 101-105 |
| 10 | 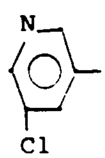 | $CH(CH_3)_2$ | 94-99 |
| 11 | 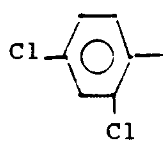 | $C(CH_3)_3$ | 126-130 |

| Nr. | Aryl | $R_3$ | Fp.[°C] |
|---|---|---|---|
| 12 | 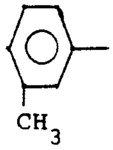 | $CH(CH_3)_2$ | 85-88 |
| 13 | 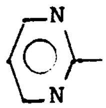 | $CH(CH_3)_2$ | 170-174 |
| 14 | 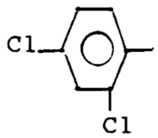 | $C_6H_5$ | 129-145 |
| 15 | 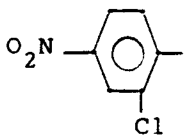 | $CH(CH_3)_2$ | 139-142 |
| 16 | 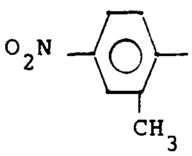 | $CH(CH_3)_2$ | 124-126 |
| 17 | 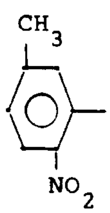 | $CH(CH_3)_2$ | 102-104 |

| Nr. | Aryl | $R_3$ | Fp.[°C] |
|---|---|---|---|
| 18 | 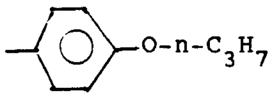 | $CH(CH_3)_2$ | 78-80 |
| 19 | 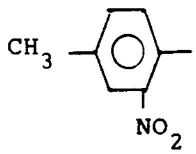 | $CH(CH_3)_2$ | 87-92 |
| 20 | 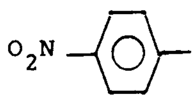 | $CH(CH_3)_2$ | 110-112 |
| 21 | 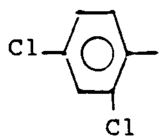 | $CH_2SC_2H_5$ | Öl |
| 22 | 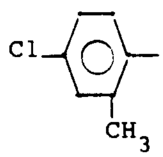 | 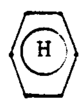 | ab 112 |
| 23 | 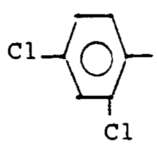 | 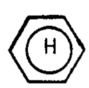 | ab 112 |

| Nr. | Aryl | $R_3$ | Fp.[°C] |
|---|---|---|---|
| 24 | 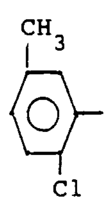 | $CH(CH_3)_2$ | 69-71 |

Tabelle VI

Verbindungen der Formel

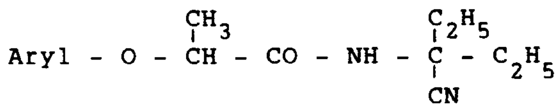

| Nr. | Aryl | Fp[°C] |
|---|---|---|
| 1 | 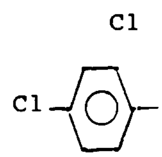 | 100-102 |
| 2 | 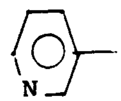 | Öl |
| 3 | 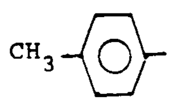 | 93-94 |
| 4 | 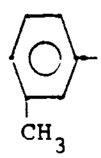 | 64-66 |
| 5 | 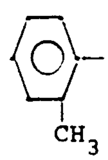 | 92-93 |
| 6 | 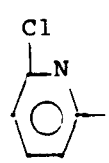 | 88-90 |

| Nr. | Aryl | Fp[°C] |
|---|---|---|
| 7 | 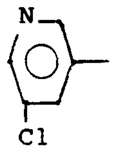 | 84-87 |
| 8 | 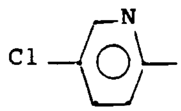 | 129-131 |
| 9 | 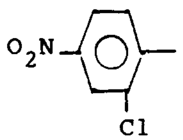 | 117-120 |
| 10 | 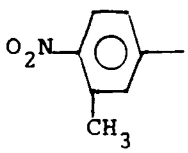 | 117-118 |
| 11 | 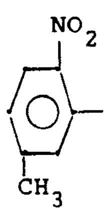 | 132-133 |
| 12 | 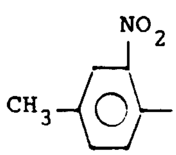 | 120-122 |
| 13 | 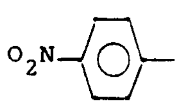 | 108-110 |
| 14 | 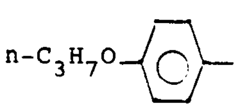 | 71-75 |

Tabelle VII

Verbindungen der Formel

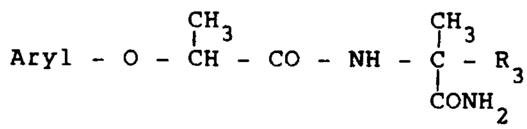

| Nr. | Aryl | $R_3$ | Fp.[°C] |
|---|---|---|---|
| 1 | 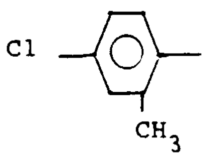 | $n-C_3H_7$ | 100-102 |
| 2 | 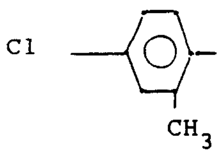 | $n-C_3H_7$ | 108-111 |
| 3 | 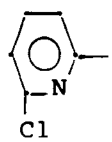 | $n-C_3H_7$ | 111-113 |
| 4 | 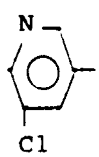 | $n-C_3H_7$ | 115-117 |

## Tabelle VIII

Verbindungen der Formal

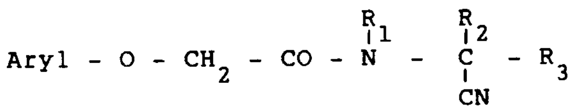

| Nr. | Aryl | $R_1$ | $R_2$ | $R_3$ | Fp.[°C] |
|---|---|---|---|---|---|
| 1 | 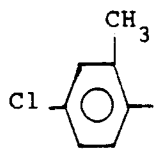 | H | $CH_3$ | $CH(CH_3)_2$ | 100-103 |
| 2 | 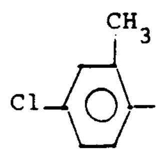 | H | $C_2H_5$ | $C_2H_5$ | 86-88 |
| 3 | 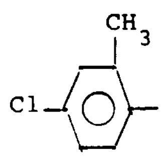 | $CH_3$ | $C_2H_5$ | $CH(CH_3)_2$ | Öl |
| 4 | 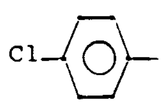 | H | $C_2H_5$ | $C_2H_5$ | 50-52 |
| 5 | 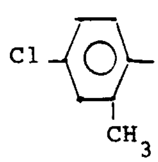 | H | $CH_3$ | $n\text{-}C_5H_{11}$ | Öl |

Tabelle IX

Weitere erfindungsgemäßen Verbindungen

| | | |
|---|---|---|
| 1 | 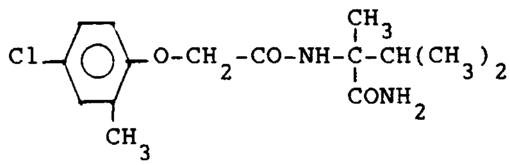 | Fp.105-107°C |
| 2 | 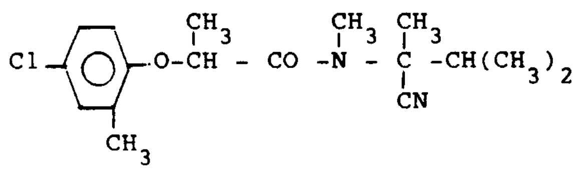 | Öl |
| 3 | 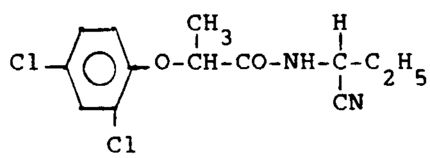 | Fp.61-63°C |
| 4 | 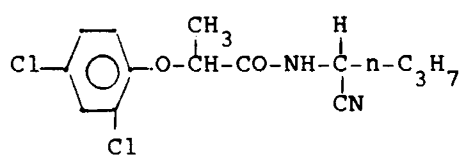 | Fp.70-74°C |

## Patentansprüche

## Patentansprüche für folgende Vertragsstaaten : BE, CH, DE, FR, GB, GR, IT, LI, LU, NL, SE

**1.** Verwendung von Verbindungen der Formel

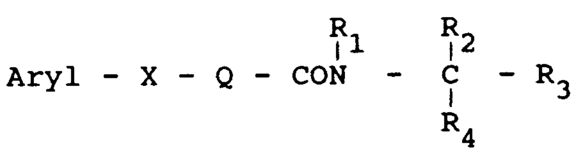 (I)

in der

Aryl: einen unsubstituierten oder ein- bis dreifach durch $C_1$-$C_5$-Alkyl, $C_1$-$C_5$-Alkoxy, $C_1$-$C_5$-Alkyl-$SO_n$ (n = 0, 1 oder 2), Halogen, $NO_2$, $CF_3$, CN, $CH_3OCH_2$, $(CH_3)_2NCH_2$, COOAlkyl, $CONH_2$ oder Phenyl substituierten Phenylrest, einen 1- oder 2-Naphthyl-, einen gegebenenfalls chlorsubstituierten 2-, 3- oder 4-Pyridyl-, einen Pyrimidyl oder Chinolylrest,

Q:

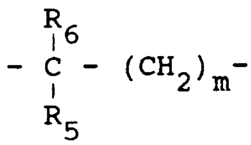

(m = 0,1 oder 2)

$R_1$: H, $C_1$-$C_5$-Alkyl, Allyl,

$R_2$ und $R_3$: H, $C_1$-$C_6$-Alkyl (das auch ein 0- oder S-Atom in der Kette enthalten kann), $C_3$-$C_7$-Cycloalkyl, $CH_2$-COO-($C_1$-$C_5$-Alkyl), Phenyl,
$R_2$ und $R_3$ gemeinsam auch $-(CH_2)_4-$,$-(CH_2)_5-$,

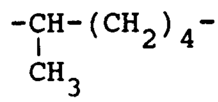

$R_4$: CN, $CONH_2$,

$R_5$: H, $CH_3$, $C_2H_5$,

$R_6$ H, $CH_3$,

X: O oder S

gegebenenfalls in Form von Racematen bzw. Gemischen der optischen Isomeren bzw. in Form der reinen Enantiomeren bzw. Diastereomeren, zur Bekämpfung von Piricularia an Reis.

**2.** Verbindungen der Formel

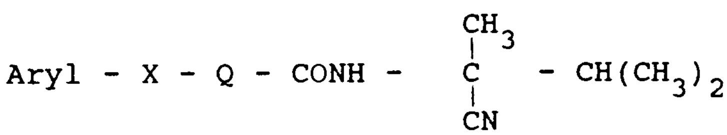 (IA)

in der

Aryl: einen unsubstituierten oder ein- bis dreifach durch $C_1$-$C_5$-Alkyl, $C_1$-$C_5$-Alkoxy, $C_1$-$C_5$-Alkyl-$SO_n$ (n = 0, 1 oder 2), Halogen, $NO_2$, $CF_3$, CN, $CH_3OCH_2$, $(CH_3)_2NCH_2$, COOAlkyl, $CONH_2$ oder Phenyl substituierten Phenylrest, einen 1- oder 2-Naphthyl-, einen gegebenenfalls chlorsubstituierten 2-, 3- oder 4-Pyridyl-, einen Pyrimidyl oder Chinolylrest,

Q:

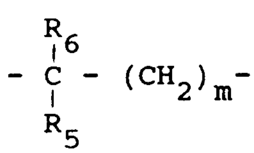

(m = 0,1 oder 2)

$R_5$: H, $CH_3$, $C_2H_5$,

$R_6$: H, $CH_3$,

X: O oder S

gegebenenfalls in Form von Racematen bzw. Gemischen der optischen Isomeren bzw. in Form der reinen Enantiomeren bzw. Diastereomeren.

**3.** Verbindungen nach Anspruch 2, worin Aryl 4-Chlorphenyl, 4-Chlor-2-methylphenyl, 2,4-, 3,4- oder 3,5-Dichlorphenyl ist.

**4.** Verbindungen nach Anspruch 2 oder 3, worin X 0 bedeutet.

**5.** Verbindungen nach Anspruch 2, 3 oder 4, worin Q $CH(CH_3)$ bedeutet.

**6.** 2-(4-Chlor-2-methylphenoxy)-propionsäure-N-[2-cyano-3-methylbutyl(2)]-amid.

**7.** Fungizide Mittel, gekennzeichnet durch einen Gehalt an einer Verbindung der Formel IA nach Anspruch 2 bis 6 gegebenenfalls in Form von Racematen bzw. Gemischen der optischen Isomeren bzw. in Form der reinen Enantiomeren bzw. Diastereomeren, neben üblichen Hilfs und/oder Trägerstoffen.

**8.** Verfahren zur Herstellung von Verbindungen nach Anspruch 2 bis 6 nach an sich bekannten Methoden, dadurch gekennzeichnet, dass man
a) eine Verbindung der Formel

Aryl - X - Z - COY (II),

worin Aryl, X und Q die obige Bedeutung haben und Y eine "leaving group" ist, mit einer Verbindung der Formel

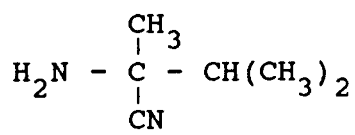
(III),

umsetzt oder dass man
b) eine Verbindung der Formel

Aryl - X - M (IV),

worin Aryl und X die obige Bedeutung haben und M Wasserstoff oder ein Alkalikation bedeutet, mit einer Verbindung der Formel

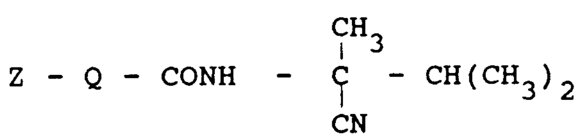
(V),

in der Q die obige Bedeutung hat und Z Halogen oder eine Arylsulfonyloxygruppe bedeutet, umsetzt und dass man gewünschtenfalls vorliegende Gemische von Enantiomeren nach üblichen Verfahren in die einzelnen Enantiomeren bzw. in Diastereomerenpaare auftrennt.

**Patentansprüche für folgende Vertragsstaaten : AT, ES**

**1.** Verwendung von Verbindungen der Formel

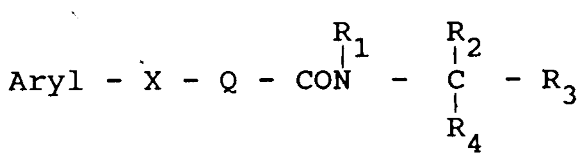
(I)

in der
Aryl: einen unsubstituierten oder ein- bis dreifach durch $C_1$-$C_5$-Alkyl, $C_1$-$C_5$-Alkoxy, $C_1$-$C_5$-Alkyl-$SO_n$ (n = 0, 1 oder 2), Halogen, $NO_2$, $CF_3$, CN, $CH_3OCH_2$, $(CH_3)_2NCH_2$, COOAlkyl, $CONH_2$ oder Phenyl substituierten Phenylrest, einen 1- oder 2-Naphthyl-, einen gegebenenfalls chlorsubstituierten 2-, 3- oder 4-Pyridyl-, einen Pyrimidyl oder

Chinolylrest,

Q:

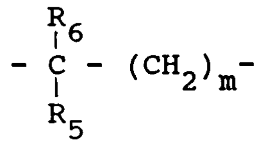

(m = 0,1 oder 2)

$R_1$: H, $C_1$-$C_5$-Alkyl, Allyl,

$R_2$ und $R_3$: H, $C_1$-$C_6$-Alkyl (das auch ein 0- oder S-Atom in der Kette enthalten kann), $C_3$-$C_7$-Cycloalkyl, $CH_2$-COO-($C_1$-$C_5$-Alkyl), Phenyl, $R_2$ und $R_3$ gemeinsam auch $-(CH_2)_4-$,$-(CH_2)_5-$,

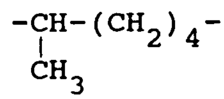

$R_4$: CN, $CONH_2$,

$R_5$: H, $CH_3$, $C_2H_5$,

$R_6$ H, $CH_3$,

X: O oder S

gegebenenfalls in Form von Racematen bzw. Gemischen der optischen Isomeren bzw. in Form der reinen Enantiomeren bzw. Diastereomeren, zur Bekampfung von Piricularia an Reis.

**2.** Verfahren zur Bekämpfung von Piricularia an Reis, welches die Behandlung der Reispflanze mit einer Verbindung der Formel

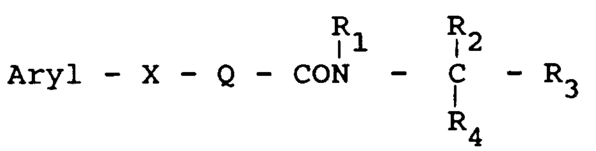

(I)

in der

Aryl: einen unsubstituierten oder ein- bis dreifach durch $C_1$-$C_5$-Alkyl, $C_1$-$C_5$-Alkoxy, $C_1$-$C_5$-Alkyl-$SO_n$ (n = 0, 1 oder 2), Halogen, $NO_2$, $CF_3$, CN, $CH_3OCH_2$, $(CH_3)_2NCH_2$, COOAlkyl, $CONH_2$ oder Phenyl substituierten Phenylrest, einen 1- oder 2-Naphthyl-, einen gegebenenfalls chlorsubstituierten 2-, 3- oder 4-Pyridyl-, einen Pyrimidyl oder Chinolylrest,

Q:

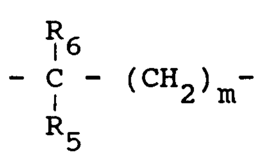

(m = 0,1 oder 2)

$R_1$: H, $C_1$-$C_5$-Alkyl, Allyl,

$R_2$ und $R_3$: H, $C_1$-$C_6$-Alkyl (das auch ein 0- oder S-Atom in der Kette enthalten kann), $C_3$-$C_7$-Cycloalkyl, $CH_2$-COO-($C_1$-$C_5$-Alkyl), Phenyl, $R_2$ und $R_3$ gemeinsam auch $-(CH_2)_4-$,$-(CH_2)_5-$,

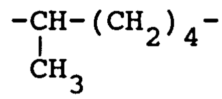

| | |
|---|---|
| $R_4$: | CN, $CONH_2$, |
| $R_5$: | H, $CH_3$, $C_2H_5$, |
| $R_6$ | H, $CH_3$, |
| X: | O oder S |

gegebenenfalls in Form von Racematen bzw. Gemischen der optischen Isomeren bzw. in Form der reinen Enantiomeren bzw. Diastereomeren, umfaßt.

**3.** Verfahren zur Herstellung von Verbindungen der Formel

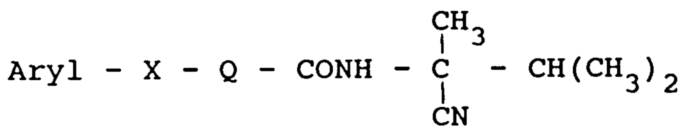 (IA)

in der

Aryl: einen unsubstituierten oder ein- bis dreifach durch $C_1$-$C_5$-Alkyl, $C_1$-$C_5$-Alkoxy, $C_1$-$C_5$-Alkyl-$SO_n$ (n = 0, 1 oder 2), Halogen, $NO_2$, $CF_3$, CN, $CH_3OCH_2$, $(CH_3)_2NCH_2$, COOAlkyl, $CONH_2$ oder Phenyl substituierten Phenylrest, einen 1- oder 2-Naphthyl-, einen gegebenenfalls chlorsubstituierten 2-, 3- oder 4-Pyridyl-, einen Pyrimidyl oder Chinolylrest,

Q:

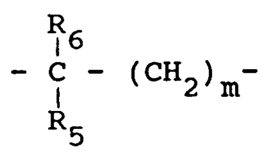

(m = 0,1 oder 2)

| | |
|---|---|
| $R_5$: | H, $CH_3$, $C_2H_5$, |
| $R_6$: | H, $CH_3$, |
| X: | O oder S |

gegebenenfalls in Form von Racematen bzw. Gemischen der optischen Isomeren bzw. in Form der reinen Enantiomeren bzw. Diastereomeren, nach an sich bekannten Methoden, dadurch gekennzeichnet, dass man

a) eine Verbindung der Formel

Aryl - X - Z - COY (II),

worin Aryl, X und Q die obige Bedeutung haben und Y eine "leaving group" ist, mit einer Verbindung der Formel

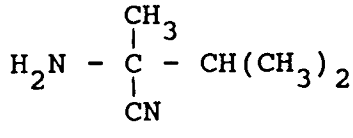 (III),

umsetzt oder dass man

b) eine Verbindung der Formel

Aryl - X - M (IV),

worin Aryl und X die obige Bedeutung haben und M Wasserstoff oder ein Alkalikation bedeutet, mit einer Verbindung der Formel

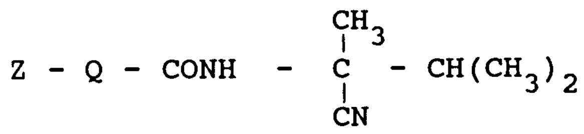

(V),

in der Q die obige Bedeutung hat und Z Halogen oder eine Arylsulfonyloxygruppe bedeutet, umsetzt

und dass man gewünschtenfalls vorliegende Gemische von Enantiomeren nach üblichen Verfahren in die einzelnen Enantiomeren bzw in Diastereomerenpaare auftrennt.

**4.** Verfahren nach Anspruch 3, worin Aryl 4-Chlorphenyl, 4-Chlor-2-methylphenyl, 2,4-, 3,4- oder 3,5-Dichlorphenyl ist.

**5.** Verfahren nach Anspruch 3 oder 4, worin X 0 bedeutet.

**6.** Verfahren nach Anspruch 3, 4 oder 5, worin Q $CH(CH_3)$ bedeutet.

**7.** Verfahren nach Anspruch 3, 4, 5 oder 6, worin die Verbindung der Formel IA ist 2-(4-Chlor-2-methylphenoxy)-propionsäure-N-[2-cyano-3-methylbutyl(2)]-amid.

**8.** Verfahren zur Herstellung von fungiziden Mitteln, gekennzeichnet durch eine Verbindung der Formel IA, wie in einem der nach Ansprüche 3 bis 7 defininiert, gegebenenfalls in Form von Racematen bzw. Gemischen der optischen Isomeren bzw. in Form der reinen Enantiomeren bzw. Diastereomeren, in Verbindung gebracht mit den üblichen Hilfs und/oder Trägerstoffen.

**Claims**

**Claims for the following Contracting States : BE, CH, DE, FR, GB, GR, IT, LI, LU, NL, SE**

**1.** Use of compounds of the formula

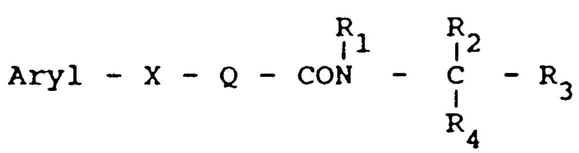

(I)

in which

Aryl: a phenyl residue unsubstituted or one- to three-fold substituted by $C_1$-$C_5$-alkyl, $C_1$-$C_5$-alkoxy, $C_1$-$C_5$-alkyl-$SO_n$ (n = 0, 1 or 2), halogen, $NO_2$, $CF_3$, CN, $CH_3OCH_2$,$(CH_3)_2NCH_2$, COO-alkyl, $CONH_2$ or phenyl, a 1- or 2-naphthyl, an optionally chlorine-substituted 2-, 3- or 4-pyridyl, a pyrimidyl or quinolyl residue,

Q:

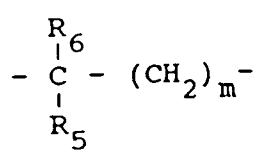

(m = 0, 1 or 2)

$R_1$: H, $C_1$-$C_5$-alkyl, allyl,

$R_2$ and $R_3$: H, $C_1$-$C_6$-alkyl (which can also contain an O or S atom in the chain), $C_3$-$C_7$-cycloalkyl, $CH_2$-COO-($C_1$-$C_5$-alkyl), phenyl,
$R_2$ and $R_3$ together also mean $-(CH_2)_4-$,$-(CH_2)_5-$,

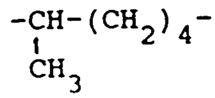

$R_4$: CN, $CONH_2$,
$R_5$: H, $CH_3$, $C_2H_5$
$R_6$: H, $CH_3$
X: O or S,

optionally in the form of racemates or mixtures of the optical isomers or in the form of the pure enantiomers or diastereomers, for the control of Piricularia on rice.

**2.** Compounds of the formula,

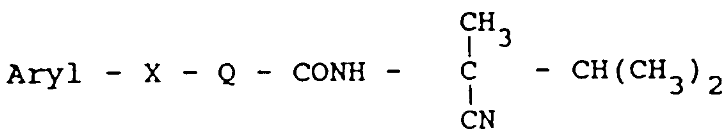 (IA)

in which

Aryl: a phenyl residue unsubstituted or one- to three-fold substituted by $C_1$-$C_5$-alkyl, $C_1$-$C_5$-alkoxy, $C_1$-$C_5$-alkyl-$SO_n$ (n = 0, 1 or 2), halogen, $NO_2$, $CF_3$, CN, $CH_3OCH_2$, $(CH_3)_2NCH_2$, COO-alkyl, $CONH_2$ or phenyl, a 1- or 2-naphthyl, an optionally chlorine-substituted 2-, 3- or 4-pyridyl, a pyrimidyl or quinolyl residue,

Q:

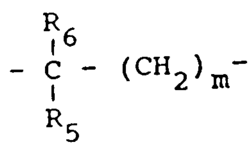

(m = 0, 1 or 2)

$R_5$: H, $CH_3$, $C_2H_5$
$R_6$: H, $CH_3$
X: O or S,

optionally in the form of racemates or mixtures of the optical isomers or in the form of the pure enantiomers or diastereomers.

**3.** Compounds according to Claim 2, wherein aryl is 4-chlorophenyl, 4-chloro-2-methylphenyl, 2,4-, 3,4- or 3,5-dichlorophenyl.

**4.** Compounds according to Claim 2 or 3, wherein X means O.

**5.** Compounds according to Claim 2, 3 or 4, wherein Q means $CH(CH_3)$.

**6.** N-[2-cyano-3-methylbutyl(2)]-2-(4-chloro-2-methylphenoxy)-propionamide.

**7.** Fungicidal agents, characterised by a content of a compound of the formula IA according to Claim 2 to 6, optionally in the form of racemates or mixtures of the optical isomers or in the form of the pure enantiomers or diastereomers, with normal additives and/or carrier substances.

**8.** Process for the preparation of compounds according to Claims 2 to 6 by known methods, characterised in that

a) a compound of the formula

Aryl - X - Q - COY (II),

where aryl, X and Q have the above meaning and Y is a leaving group, is reacted with a compound of the formula

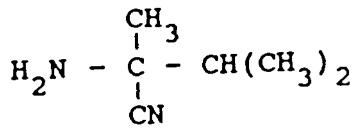 (III),

or that

b) a compound of the formula

Aryl - X - M (IV),

where aryl and X have the above meaning, and M means hydrogen or an alkali metal cation, is reacted with a compound of the formula

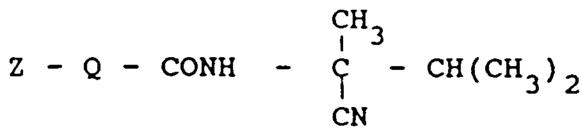 (V),

in which Q has the above meaning and Z means halogen or an arylsulphonyloxy group and that if desired mixtures of enantiomers present are separated by normal methods into the individual enantiomers or into diastereomer pairs.

**Claims for the following Contracting States : AT, ES**

**1.** Use of compounds of the formula I

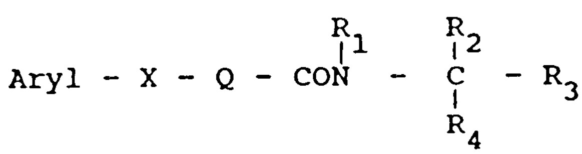 (I)

in which

Aryl: a phenyl residue unsubstituted or one- to three-fold substituted by $C_1$-$C_5$-alkyl, $C_1$-$C_5$-alkoxy, $C_1$-$C_5$-alkyl-$SO_n$ (n = 0, 1 or 2), halogen, $NO_2$, $CF_3$, CN, $CH_3OCH_2$, $(CH_3)_2NCH_2$, COO-alkyl, $CONH_2$ or phenyl, a 1- or 2-naphthyl, an optionally chlorine-substituted 2-, 3- or 4-pyridyl, a pyrimidyl or quinolyl residue,

Q:

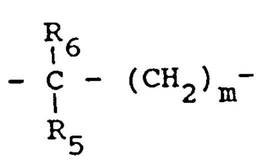

(m = 0, 1 or 2)
$R_1$: H, $C_1$-$C_5$-alkyl, allyl,
$R_2$ and $R_3$: H, $C_1$-$C_6$-alkyl (which can also contain an O or S atom in the chain), $C_3$-$C_7$-cycloalkyl, $CH_2$-COO-($C_1$-$C_5$-alkyl), phenyl,
$R_2$ and $R_3$ together also mean $-(CH_2)_4-$,$-(CH_2)_5-$,

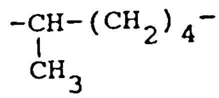

$R_4$: CN, $CONH_2$,
$R_5$: H, $CH_3$, $C_2H_5$
$R_6$: H, $CH_3$,
X: O or S,

optionally in the form of racemates or mixtures of the optical isomers or in the form of the pure enantiomers or diastereomers, for the control of Piricularia on rice.

**2.** Process for the control of Piricularia on rice, which involves the treatment of the rice plants with a compound of the formula

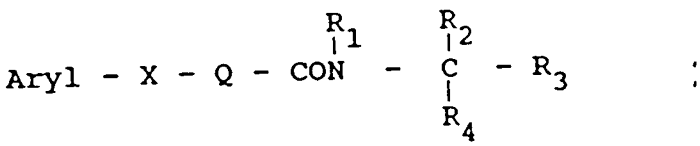

(I)

in which
Aryl: a phenyl residue unsubstituted or one- to three-fold substituted by $C_1$-$C_5$-alkyl, $C_1$-$C_5$-alkoxy, $C_1$-$C_5$-alkyl-$SO_n$ (n = 0, 1 or 2), halogen, $NO_2$, $CF_3$, CN, $CH_3OCH_2$, $(CH_3)_2NCH_2$, COO-alkyl, $CONH_2$ or phenyl, a 1- or 2-naphthyl, an optionally chlorine-substituted 2-, 3- or 4-pyridyl, a pyrimidyl or quinolyl residue,
Q:

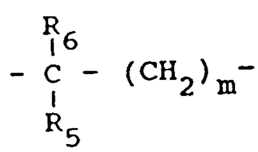

(m = 0, 1 or 2)
$R_1$: H, $C_1$-$C_5$-alkyl, allyl,
$R_2$ and $R_3$: H, $C_1$-$C_6$-alkyl (which can also contain an O or S atom in the chain), $C_3$-$C_7$-cycloalkyl, $CH_2$-COO-($C_1$-$C_5$-alkyl), phenyl,
$R_2$ and $R_3$ together also mean $-(CH_2)_4-$,$-(CH_2)_5-$

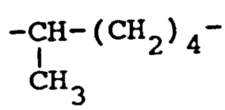

$R_4$: CN, $CONH_2$,
$R_5$: H, $CH_3$, $C_2H_5$
$R_6$: H, $CH_3$,
X: O or S,

optionally in the form of racemates or mixtures of the optical isomers or in the form of the pure

enantiomers or diastereomers.

**3.** Process for the preparation of compounds of the formula

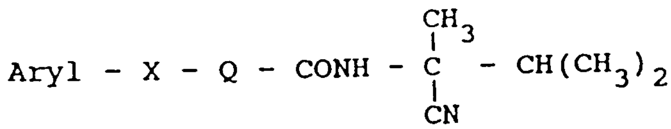 (IA)

in which

Aryl: a phenyl residue unsubstituted or one- to three-fold substituted by $C_1$-$C_5$-alkyl, $C_1$-$C_5$-alkoxy, $C_1$-$C_5$-alkyl-$SO_n$ (n = 0, 1 or 2), halogen, $NO_2$, $CF_3$, CN, $CH_3OCH_2$, $(CH_3)_2NCH_2$, COO-alkyl, $CONH_2$ or phenyl, a 1- or 2-naphthyl, an optionally chlorine-substituted 2-, 3- or 4-pyridyl, a pyrimidyl or quinolyl residue,

Q:

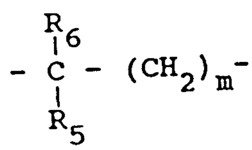

(m = 0, 1 or 2)

$R_5$: H, $CH_3$, $C_2H_5$

$R_6$: H, $CH_3$

X: O or S,

optionally in the form of racemates or mixtures of the optical isomers or in the form of the pure enantiomers or diastereomers by known methods, characterised in that

a) a compound of the formula

Aryl - X - Q - COY (II),

where aryl, X and Q have the above meaning and Y is a leaving group, is reacted with a compound of the formula

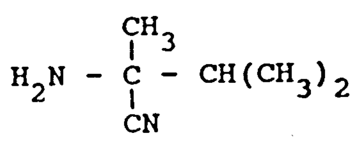 (III),

or that

b) a compound of the formula

Aryl - X - M (IV),

where aryl and X have the above meaning, and M means hydrogen or an alkali metal cation, is reacted with a compound of the formula

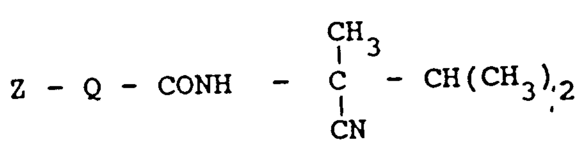 (V),

in which Q has the above meaning and Z means halogen or an arylsulphonyloxy group and that if desired mixtures of enantiomers present are separated by normal methods into the individual enantiomers or into diastereomer pairs.

**4.** Process according to Claim 3, wherein aryl is 4-chlorophenyl, 4-chloro-2-methylphenyl, 2,4-, 3,4- or 3,5-dichlorophenyl.

**5.** Process according to Claim 3 or 4, wherein X means O.

**6.** Process according to Claim 3, 4 or 5, wherein Q means $CH(CH_3)$.

**7.** Process according to Claim 3, 4, 5 or 6, wherein the compound of the formula IA is N-[2-cyano-3-methylbutyl(2)]-2-(4-chloro-2-methylphenoxy)-propionamide.

**8.** Process for the preparation of fungicidal agents, characterised by a compound of the formula IA, as defined in one of the Claims 3 to 7, optionally in the form of racemates or mixtures of the optical isomers or in the form of the pure enantiomers or diastereomers, combined with the normal additives and/or carrier substances.

**Revendications**

**Revendications pour les Etats contractants suivants : BE, CH, DE, FR, GB, GR, IT, LI, LU, NL, SE**

**1.** Utilisation des composés de formule

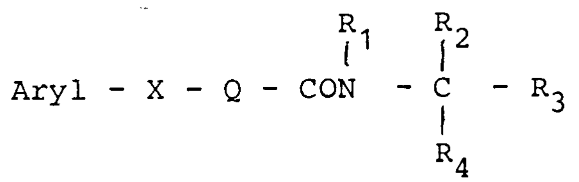

(I)

dans laquelle

aryle représente un reste phényle non substitué ou substitué une à trois fois par un alkyle en $C_1$-$C_5$, par un alcoxy en $C_1$-$C_5$, par un alkyle en $C_1$-$C_5$ $SO_n$ (n = 0, 1 ou 2), par un halogène, par $NO_2$, $CF_3$, CN, $CH_3OCH_2$, $(CH_3)_2NCH_2$, COOalkyle, $CONH_2$ ou par le phényle, ou un reste 1-naphtyle ou 2-naphtyle, un reste 2-pyridyle, 3-pyridyle ou 4-pyridyle éventuellement substitué par le chlore, un reste pyrimidyle ou un reste quinolyle,

Q

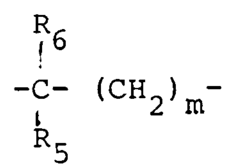

(m = 0, 1 ou 2)

$R_1$ H, un alkyle en $C_1$-$C_5$, un allyle,

$R_2$ et $R_3$ H, un alkyle en $C_1$-$C_6$ (pouvant également contenir un atome d'O ou de S dans la chaîne), un cycloalkyle en $C_3$-$C_7$, $CH_2$-COO-(alkyle en $C_1$-$C_5$), un phényle,

$R_1$ et $R_3$ représentent ensemble également $-(CH_2)_4-$, $-(CH_2)_5-$,

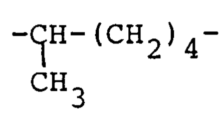

$R_4$ CN, $CONH_2$,
$R_5$ H, $CH_3$, $C_2H_5$,
$R_6$ H, $CH_3$,
X O ou S

éventuellement sous forme de racémates ou de mélanges des isomères optiques ou sous forme d'énantiomères ou de diastéréoisomères purs pour lutter contre le Piricularia sur le riz.

**2.** Composés de formule

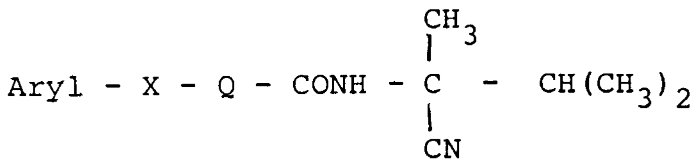

(IA)

dans laquelle

aryle représentant un reste phényle non substitué ou substitué une ou plusieurs fois par un alkyle en $C_1$-$C_5$, par un alcoxy en $C_1$-$C_5$, par un alkyle en $C_1$-$C_5$ $SO_n$ (n = 0, 1 ou 2), par un halogène, par $NO_2$, $CF_3$, CN, $CH_3OCH_2$, $(CH_3)_2NCH_2$, COOalkyle, $CONH_2$ ou un phényle, un reste 1-naphtyle ou 2-naphtyle, un reste 2-pyridyle, 3-pyridyle ou 4-pyridyle, éventuellement substitué par le chlore, un reste pyrimidyle ou un reste quinolyle

Q

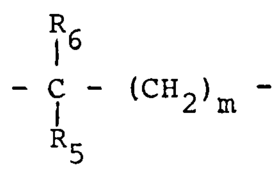

(m = 0, 1 ou 2)

$R_5$ H, $CH_3$, $C_2H_5$,
$R_6$ H, $CH_3$
X O ou S

éventuellement sous forme de racémates ou de mélanges d'isomères optiques ou sous forme d'énantiomères ou de diastéréoisomères purs.

**3.** Composés selon la revendication 2 où l'aryle est le 4-chlorophényle, le 4-chloro-2-méthylphényle, le 2,4-, 3,4- ou 3,5-dichlorophényle.

**4.** Composés selon la revendication 2 ou 3 où X représente O.

**5.** Composés selon les revendications 2, 3 ou 4 où Q représente $CH(CH_3)$.

**6.** N-[2-cyano-3-méthylbutyl(2)]-amide de l'acide 2-(4-chloro-2-méthylphénoxy)-propionique.

**7.** Agents fongicides, caractérisés en ce qu'ils contiennent un composé de formule IA selon les revendications 2 à 6, éventuellement sous forme de racémates ou de mélanges d'isomères optiques ou sous forme d'énantiomères ou de diastéréoisomères purs, à côté d'adjuvants et/ou de supports usuels.

**8.** Procédé pour la préparation des composés selon les revendications 2 à 6 selon des méthodes connues en soi, caractérisé

a) en ce que l'on fait réagir un composé de formule

Aryl - X - Z - COY (II)

où aryle, X et Q ont les significations données ci-dessus et Y est un "leaving group" avec un

composé de formule

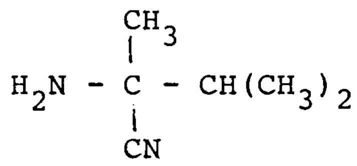

(III)

ou

b) en ce que l'on fait réagir un composé de formule

aryl - X - M (IV)

où aryle et X ont les significations données ci-dessus et M représente l'hydrogène ou un cation alcalin avec un composé de formule

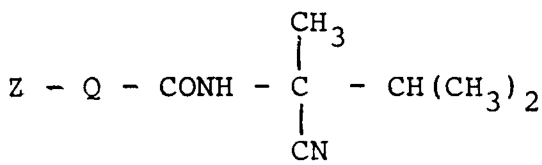

(V)

dans laquelle Q a la signification donnée ci-dessus et Z représente un halogène ou un groupe arylsulfonyloxy, et en ce que l'on sépare, si désiré, les mélanges d'énantiomères se présentant selon des procédés usuels en énantiomères isolés ou en couples de diastéréoisomères.

**Revendications pour les Etats contractants suivants : AT, ES**

**1.** Utilisation des composés de formule

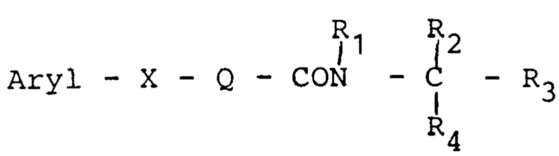

(I)

dans laquelle

aryle représente un reste phényle non substitué ou substitué une à trois fois par un alkyle en $C_1$-$C_5$, par un alcoxy en $C_1$-$C_5$, par un alkyle en $C_1$-$C_5$ $SO_n$ (n = 0, 1 ou 2), par un halogène, par $NO_2$, $CF_3$, CN, $CH_3OCH_2$, $(CH_3)_2NCH_2$, COOalkyle, $CONH_2$ ou par le phényle, ou un reste 1-naphtyle ou 2-naphtyle, un reste 2-pyridyle, 3-pyridyle ou 4-pyridyle éventuellement substitué par le chlore, un reste pyrimidyle ou un reste quinolyle,

Q

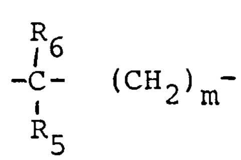

(m = 0, 1 ou 2)

$R_1$ H, un alkyle en $C_1$-$C_5$, un allyle,

$R_2$ et $R_3$ H, un alkyle en $C_1$-$C_6$ (pouvant également contenir un atome d'O ou de S dans la chaîne), un cycloalkyle en $C_3$-$C_7$, $CH_2$-COO-(alkyle en $C_1$-$C_5$), un phényle,

$R_1$ et $R_3$ représentent ensemble également $-(CH_2)_4-$, $-(CH_2)_5-$,

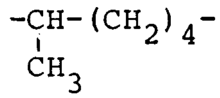

| | |
|---|---|
| $R_4$ | CN, $CONH_2$, |
| $R_5$ | N, $CH_3$, $C_2H_5$, |
| $R_6$ | H, $CH_3$, |
| X | O ou S |

éventuellement sous forme de racémates ou de mélanges des isomères optiques ou sous forme d'énantiomères ou de diastéréoisomères purs pour lutter contre le Piricularia sur le riz.

**2.** Procédé pour lutter contre le Piricularia sur le riz comprenant le traitement des plants de riz avec un composé de formule

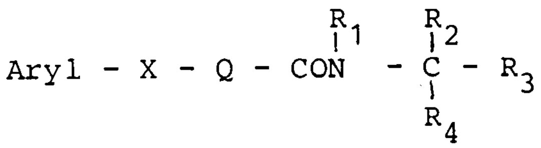

(I)

dans laquelle

aryle représente un reste phényle non substitué ou substitué une à trois fois par un alkyle en $C_1$-$C_5$, par un alcoxy en $C_1$-$C_5$, par un alkyle en $C_1$-$C_5$ $SO_n$ (n = 0, 1 ou 2), par un halogène, par $NO_2$, $CF_3$, CN, $CH_3OCH_2$, $(CH_3)_2NCH_2$, COOalkyle, $CONH_2$ ou par le phényle, ou un reste 1-naphtyle ou 2-naphtyle, un reste 2-pyridyle, 3-pyridyle ou 4-pyridyle éventuellement substitué par le chlore, un reste pyrimidyle ou un reste quinolyle,

Q

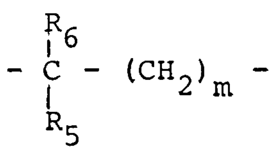

(m = 0, 1 ou 2)

$R_1$ H, un alkyle en $C_1$-$C_5$, un allyle,

$R_2$ et $R_3$ H, un alkyle en $C_1$-$C_6$ (pouvant également contenir un atome d'O ou de S dans la chaîne), un cycloalkyle en $C_3$-$C_7$, $CH_2$-COO-(alkyle en $C_1$-$C_5$), un phényle,

$R_1$ et $R_3$ représentent ensemble également $-(CH_2)_4-$, $-(CH_2)_5-$,

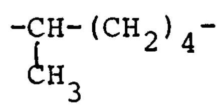

| | |
|---|---|
| $R_4$ | CN, $CONH_2$, |
| $R_5$ | H, $CH_3$, $C_2H_5$, |
| $R_6$ | H, $CH_3$, |
| X | O ou S |

éventuellement sous forme de racémates ou de mélanges des isomères optiques ou sous forme d'énantiomères ou de diastéréoisomères purs.

**3.** Procédé pour la préparation des composés de formule

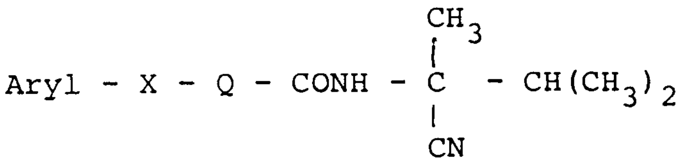 (IA)

dans laquelle

aryle représente un reste phényle non substitué ou substitué une ou plusieurs fois par un alkyle en $C_1$-$C_5$, par un alcoxy en $C_1$-$C_5$, par un alkyle en $C_1$-$C_5$ $SO_n$ (n = 0, 1 ou 2), par un halogène, par $NO_2$, $CF_3$, CN, $CH_3OCH_2$, $(CH_3)_2NCH_2$, COOalkyle, $CONH_2$ ou un phényle, un reste 1-naphtyle ou 2-naphtyle, un reste 2-pyridyle, 3-pyridyle ou 4-pyridyle, éventuellement substitué par le chlore, un reste pyrimidyle ou un reste quinolyle,

Q

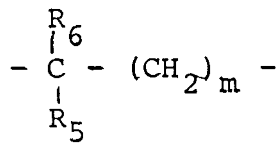

(m = O, 1 ou 2)

$R_5$ H, $CH_3$, $C_2H_5$,

$R_6$ H, $CH_3$,

X O ou S

éventuellement sous forme de racémates ou de mélanges d'isomères optiques ou sous forme d'énantiomères ou de diastéréoisomères purs, selon des méthodes connues en soi, caractérisé

a) en ce que l'on fait réagir un composé de formule

aryl - X - Z - COY (II)

où aryle, X et Q ont les significations données ci-dessus et Y est un "leaving group" avec un composé de formule

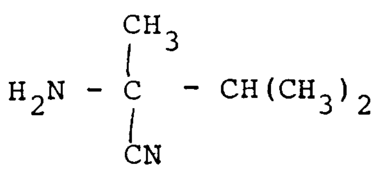 (III),

ou

b) en ce que l'on fait réagir un composé de formule

aryl - X - M (IV)

où aryle et X ont les significations données ci-dessus et M représente l'hydrogène ou un cation alcalin avec un composé de formule

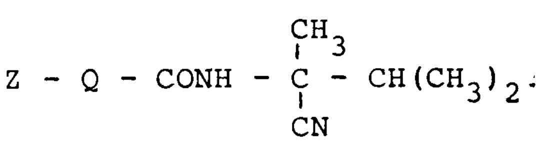 (V),

dans laquelle Q a la signification donnée ci-dessus et Z représente un halogène ou un groupe arylsulfonyloxy, et en ce que l'on sépare, si désiré, les mélanges d'énantiomères se présentant

selon des procédés usuels en énantiomères isolés ou en couples de diastéréoisomères.

**4.** Procédé selon la revendication 3 où l'aryle est le 4-chlorophényle, le 4-chloro-2-méthylphényle, le 2,4-, 3,4- ou 3,5-dichlorophényle.

**5.** Procédé selon la revendication 3 ou 4 où X représente O.

**6.** Procédé selon les revendications 3, 4 ou 5 où Q représente $CH(CH_3)$.

**7.** Procédé selon les revendications 3, 4, 5 ou 6 où le composé de formule IA est le N-[2-cyano-3-méthyl-butyl-(2)]-amide de l'acide 2-(4-chloro-2-méthylphénoxy)-propionique.

**8.** Procédé pour la préparation d'agents fongicides, caractérisé en ce que l'on associe un composé de formule IA tel que défini dans l'une des revendications 3 à 7, éventuellement sous forme de racémates ou de mélanges d'isomères optiques ou sous forme d'énantiomères ou de diastéréoisomères purs à des adjuvants et/ou supports usuels.